Europäisches Patentamt

(19) European Patent Office

Office européen des brevets

(11) Publication number: **0 102 824**
**B1**

(12) **EUROPEAN PATENT SPECIFICATION**

(45) Date of publication of patent specification: **02.12.87**

(21) Application number: **83305009.9**

(22) Date of filing: **31.08.83**

(51) Int. Cl.⁴: **F 04 B 17/04,** F 04 B 3/00, A 61 M 5/14

(54) Low power electromagnetic pump.

(30) Priority: **07.09.82 US 415657**
**23.05.83 US 496822**

(43) Date of publication of application:
**14.03.84 Bulletin 84/11**

(45) Publication of the grant of the patent:
**02.12.87 Bulletin 87/49**

(84) Designated Contracting States:
**AT BE CH DE FR GB IT LI LU NL SE**

(56) References cited:
**WO-A-81/00888**
**DE-A-2 315 842**
**GB-A- 685 143**
**GB-A- 687 756**
**GB-A-1 496 147**
**US-A-3 572 980**

(73) Proprietor: **GREATBATCH ENTERPRISES, INC.**
**10,000 Wehrle Drive**
**Clarence New York 14031 (US)**

(72) Inventor: **Falk, Theodore J.**
**10880 Boyd Drive**
**Clarence New York 14031 (US)**
Inventor: **Morris, Lawrence E.**
**10 Maple Drive**
**Bowmansville New York 14026 (US)**

(74) Representative: **Brown, David Alan et al**
**MATHYS & SQUIRE 10 Fleet Street**
**London EC4Y 1AY (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# 0 102 824

**Description**

Technical field

This invention relates to the art of electromagnetically-operated fluid pumps, and more particularly to a new and improved electromagnetic pump which operates at extremely low power.

Background art

One area of use of the present invention is implantable drug delivery systems, although the principles of the present invention can be variously applied. The principal requirements for a pump in such applications are low power drain, since the pump must be driven by an implanted battery, and compatibility with the drug being pumped. Other important requirements are that the pump be relatively insensitive to the presence of bubbles in the fluid being handled, be relatively easy to prime, and provide an adequate fluid pressure increase across the pump which is sufficient for the intended uses of the pump. A further requirement is to have low pressure drop across the pump check valve while at the same time providing satisfactory long term sealing against back flow.

It would, therefore, be highly desirable to provide an electromagnetically-operated pump which is safe, reliable, small in size, light in weight, which operates without excessive demand on the available energy supply, which is compatible with drugs of similar liquids to be pumped, which is relatively insensitive to the presence of bubbles in the fluid being pumped and relatively easy to prime, which provides an adequate fluid pressure increase across the pump sufficient for the uses intended, and which allows low pressure drop across the pump check valve while at the same time provides satisfactory long term sealing against back flow.

Published PCT application WO 81/00888 (McMullen) describes an electromagnetic pump comprising a housing having an interior fluid containing region, an inlet and an outlet in fluid communication therewith, check valve means for allowing fluid flow in a direction from the inlet to the outlet and blocking fluid flow in a direction from the outlet to the inlet, electromagnet means carried by the housing, and an armature positioned in the fluid containing region having a pole portion located for magnetic attraction by the electromagnetic means and having a piston for forcing fluid from the fluid containing region through the outlet when the armature is moved by the electromagnet means through a forward pumping stroke. The armature has a pole face separated from the electromagnet means by a gap which is closed when the armature is moved in response to energisation of the electromagnetic means. In this pump the electromagnetic means includes a fixed armature having a bore through which the fluid flows. The electromagnet means is therefore not separated from the fluid containing region.

The electromagnetic pump of the present invention is characterised in that it has a fluid containing region and an electromagnet means in axially spaced relation along the longitudinal axis of the housing and separated by barrier means of fluid impervious material disposed substantially perpendicular to the longitudinal axis of the housing, the fluid containing region and the inlet, outlet and armature of the pump all being located axially on one side of the barrier means and the electromagnet means being located axially on the opposite side of the barrier means.

In one form of the invention an electromagnetic pump comprises a housing having fluid receiving and pumping chambers therein and in communication with an inlet and an outlet, respectively, electromagnet means carried by the housing located external to the fluid chambers thereof, and barrier means in the form of a thin diaphragm of fluid impermeable material which hermetically isolates the electromagnet from the fluid chamber. An armature in the housing is movable within a body of magnetically permeable material and has a pole portion located for magnetic attraction by the electromagnet and has a plunger portion in the pumping chamber for forcing fluid out of the chamber through the outlet. The armature is moved from a rest position through a forward pumping stroke when attracted by the electromagnet means to force fluid out of the pumping chamber through the outlet, and the armature is moved by biasing means in an opposite direction through a return stroke back to the rest position. A magnetic circuit is defined including the electromagnet means, a portion of the fluid-impermeable barrier, the body, the armature pole position and a gap defined between the pole portion and the electromagnet which gap is closed during movement of the armature toward the electromagnet during energization thereof. The pump is made electrically and magnetically efficient by minimizing the total gap within the magnetic circuit, by having the pole face area relatively large on the armature pole portion, and by having the electromagnet include a coil or a core of relatively small diameter. A pump check valve can be provided separately or incorporated integrally with the armature in the form of a valve member movably carried by the armature and positioned for closing the pump inlet when the armature is in the rest position and opening the inlet after the armature begins the forward pumping stroke. In another aspect of the invention, the pump includes a pair of serially-connected fluid pumping chambers, one in communication with the fluid receiving chamber and the other in communication with the outlet, and the armature has first and second plunger portions in respective ones of the pumping chambers for forcing fluid out of the chambers and through the outlet. During the forward pumping stroke, the armature forces fluid from one pumping chamber to the other and then out of that chamber through the outlet. A pump check valve is within the pump and associated with the armature in the form of a valve member located in the fluid receiving chamber, movably carried by the armature, and positioned for closing the pump inlet when the armature is in the rest position and opening the inlet after the armature begins the forward pumping stroke. The arrangement is such that the volume of the fluid receiving chamber is minimized in the region between the check valve and the neighboring one of the armature plunger portions. Long term sealing against back flow is provided by a relatively stronger biasing means, and short term sealing by a relatively weaker biasing means.

Brief description of drawings

Figure 1 is a side elevational view of a pump according to one embodiment of the present invention;

Figure 2 is an enlarged longitudinal section view, partly in elevation, of the pump of Figure 1;

Figure 3 is a fragmentary longitudinal view of the pump of Figure 2 showing the armature in a rest position;

Figure 4 is a sectional view taken about on line 4—4 in Figure 2;

Figure 5 is a sectional view taken about on line 5—5 in Figure 2;

Figure 6 is a sectional view taken about on line 6—6 in Figure 2;

Figure 7 is a sectional view taken about on line 7—7 in Figure 2;

Figure 8 is a longitudinal sectional view of a pump according to another embodiment of the present invention;

Figure 9 is a fragmentary longitudinal sectional view of the pump of Figure 8 showing the armature in a rest position;

Figure 10 is a fragmentary longitudinal sectional view of the pump of Figure 8 showing the armature in an energized position and the check valve in a closed position;

Figure 11 is a sectional view taken about on line 11—11 in Figure 8;

Figure 12 is a sectional view taken about on line 12—12 in Figure 8;

Figure 13 is a sectional view taken about on line 13—13 in Figure 8;

Figure 14 is a sectional view taken about on line 14—14 in Figure 8;

Figure 15 is a fragmentary longitudinal, sectional view showing the pump of Figure 8 provided with an alternative form of check valve shown in an open position;

Figure 16 is a fragmentary longitudinal view similar to Figure 15 showing the check valve in a closed position;

Figure 17 is a sectional view taken about on line 17—17 in Figure 15;

Figure 18 is a sectional view taken about on line 18—18 in Figure 15;

Figure 19 is a side elevational view of a pump according to another embodiment of the present invention;

Figure 20 is an enlarged longitudinal sectional view of the pump of Figure 19 showing the armature in an energized position and the check valve in an open position;

Figure 21 is a fragmentary longitudinal sectional view of the pump of Figure 20 showing the armature in a rest position;

Figure 22 is a fragmentary longitudinal sectional view of the pump of Figure 20 showing the armature in an energized position and the check valve in a closed position;

Figure 23 is a sectional view taken about on line 23—23 in Figure 20;

Figure 24 is a sectional view taken about on line 24—24 in Figure 20;

Figure 25 is a sectional view taken about on line 25—25 in Figure 20; and

Figure 26 is a sectional view taken about on line 26—26 in Figure 20.

Best mode for carrying out the invention

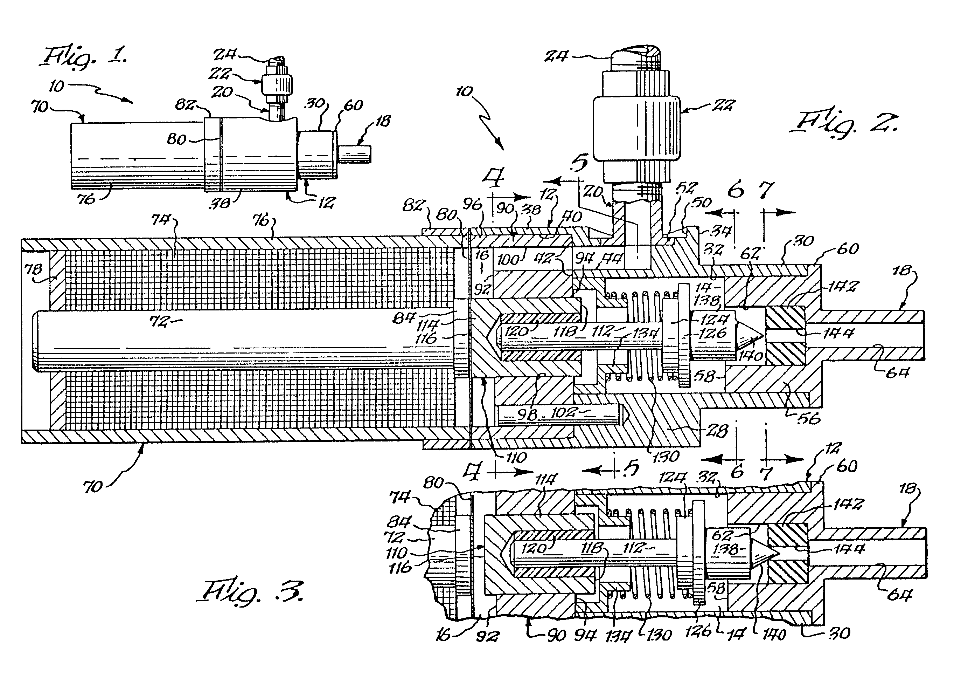

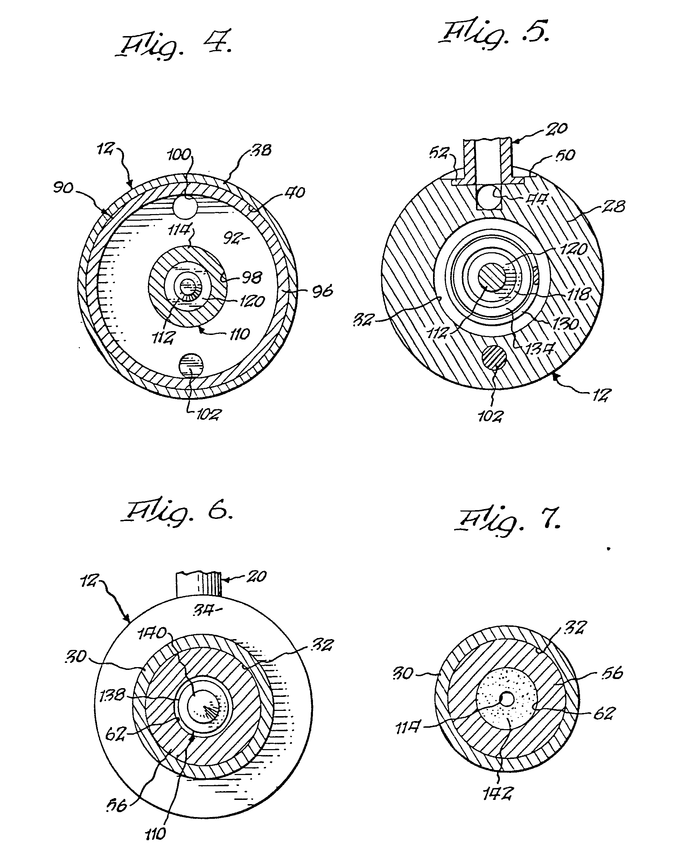

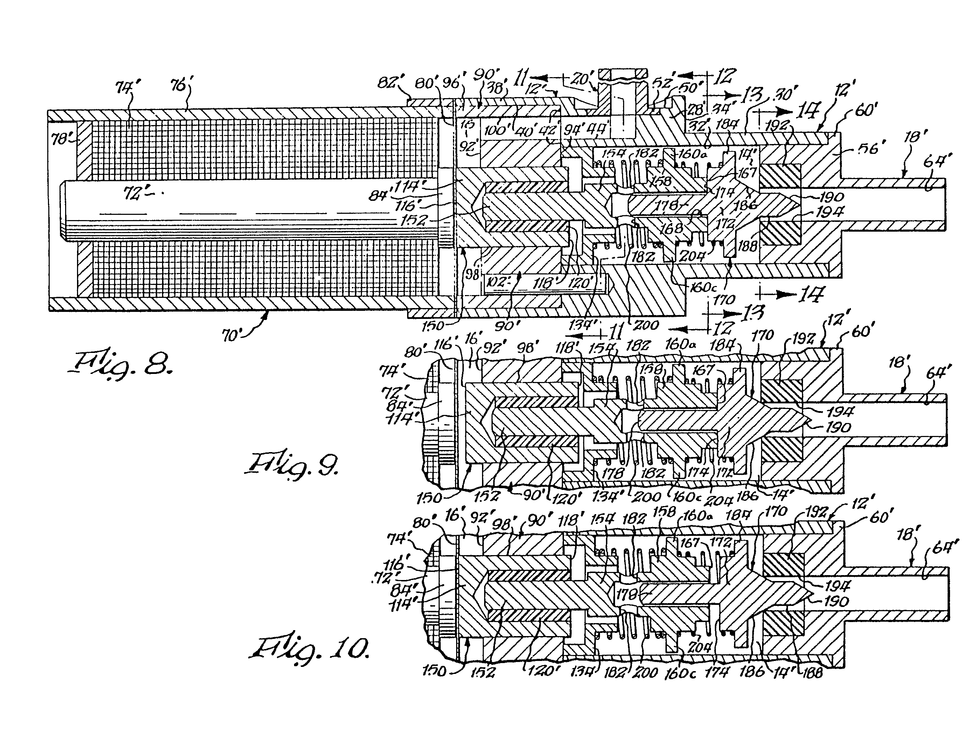

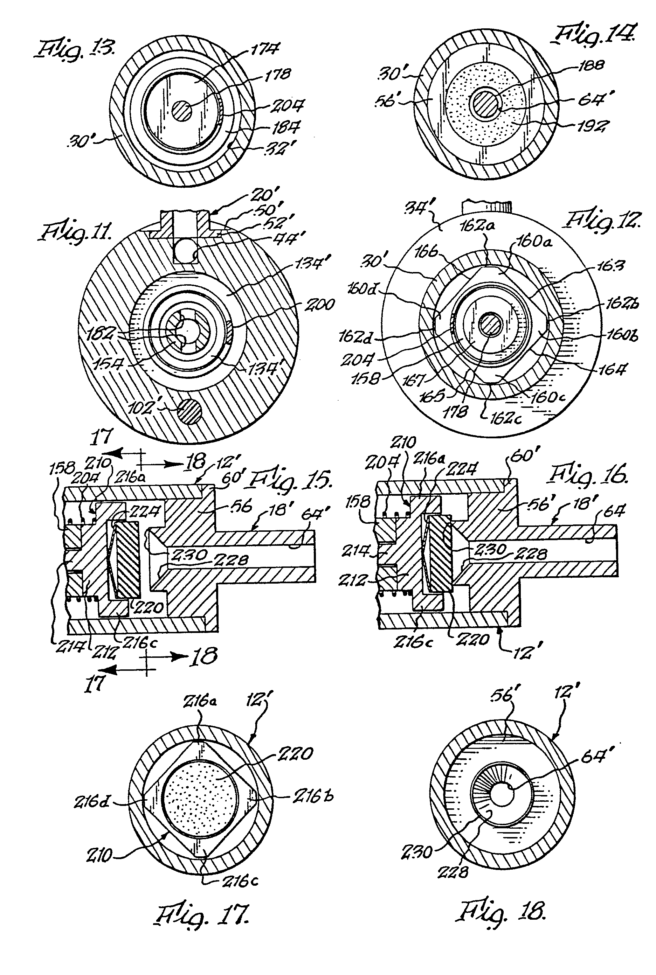

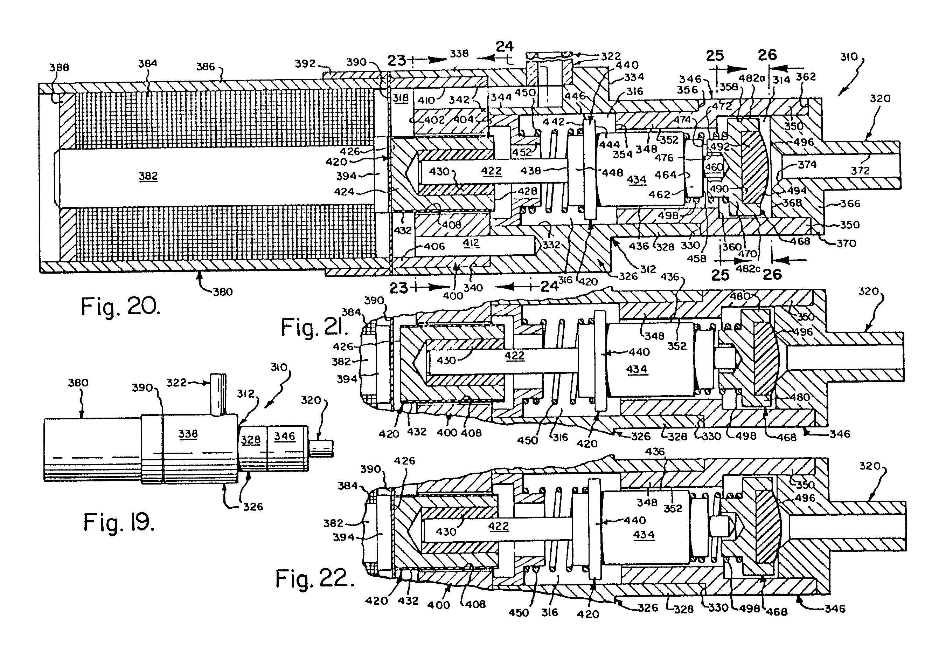

Referring now to Figures 1—7, a pump 10 according to one embodiment of the present invention includes a housing 12 which is generally hollow cylindrical in overall shape and includes an interior region for containing fluid, i.e. the liquid to be pumped. As shown in Figure 2, the hollow interior region is divided in a manner which will be described into a fluid receiving chamber 14 and a fluid pumping chamber 16 in fluid communication therewith. There is an inlet generally designated 18 in fluid communication with the receiving chamber 14 and adapted to be connected to a source or supply of fluid to be pumped. There is also an outlet 20 in fluid communication with the pumping chamber 16 and adapted to be in fluid communication with a location to which the fluid is to be pumped.

There is a check valve means operatively associated with the fluid containing region of pump 10 for allowing fluid flow in a direction from the inlet 18 through outlet 20 and blocking fluid flow in a direction from the outlet through the inlet. In the pump of this embodiment a check valve 22 is connected between outlet 20 and a conduit 24 for supplying pumped fluid to a location of use.

Referring now to Figure 2, housing 12 is generally hollow cylindrical in overall shape including a central body portion 28 of relatively substantial wall thickness. Housing 12 also includes a first axial end portion 30 extending from one end of body portion 28, i.e. the right-hand end as viewed in Figure 2, and which is of relatively smaller wall thickness. Portions 28 and 30 define the interior region of constant diameter having an inner surface 32. Thus, the outer surfaces of portions 28 and 30 are of different diameters and meet in an annular surface 34. The housing 12 has a second axial end portion 38 extending from the other end of body 28, i.e. the left-hand end as viewed in Figure 2, which also is of smaller wall thickness. End portion 38 has a relatively larger diameter inner wall surface 40 which meets surface 32 in annular intermediate wall 42.

3

Body portion 28 is provided with a longitudinal bore or passage 44 for placing pumping chamber 16 in fluid communication with outlet 20. Outlet 20 is located on the side of housing 12 for communication with the passage 44. Housing portion 28 is provided with a radially extending opening in the outer wall thereof in communication with passage 44. The opening is formed to include an annular recess 50 defining a ledge which engages an annular rim 52 of a tubular fitting which defines the outlet 20.

Inlet 18 is provided by a plug-like element which is fitted into the open end of the housing axial end portion 30. The element includes a main body 56 generally cylindrical in shape and having an outer diameter substantially equal to the diameter of inner wall surface 32 thereby providing a close fit therein. Body 56 has one axial end face 58 located in chamber 14, and adjacent the opposite end face the outer surface of body 56 is provided with an annular rim 60 which abuts the annular end face of housing portion 30 for placement and securement of the fitting in the housing. The two components are secured together by welding or other suitable means. The body portion 56 has an internal wall surface 62 defining a region of substantial diameter. The inlet portion 18 is defined by a relatively smaller diameter portion of the plug element having an internal passage 64 of relatively smaller diameter compared to passage 62. Inlet 18 is adapted for connection to a conduit such as a flexible tubing leading from a source or supply of fluid to be pumped.

By way of example, in an illustrative pump, housing 12 and the inlet and outlet fittings 18 and 20, respectively, all are of metal, and for a drug delivery pump for implantation in a patient, titanium has been found to provide satisfactory results. In such an illustrative pump, housing 12 has an overall length of about 10.8 mm (0.425 inch) measured between the axial end faces of portions 30 and 38. Surface 32 has a diameter of about 3.8 mm (0.15 inch), and the axial end face of portion 30 has a radial dimension of about 0.76 mm (0.03 inch). Surface 40 has a diameter of about 6.6 mm (0.26 inch), and the axial end face of housing portion 38 has a radial thickness of about 0.51 mm (0.02 inch). Passage 44 in housing body 28 and the interior passage in outlet fitting 20 both have a diameter of about 0.813 mm (0.032 inch). In the inlet plug fitting 18, the passage 62 has a diameter of about 1.85 mm (0.073 inch) and passage 64 has a diameter of about 0.813 mm (0.032) inch.

The pump of the present invention further comprises electromagnet means generally designated 70 carried by housing 12 and located external to the fluid containing region of the housing. As shown in Figure 2, the electromagnet 70 includes a core 72 in the form of a spool which is generally solid cylindrical in shape. A coil 74 is wound on spool 72 and contained within a hollow housing 76 generally cylindrical in shape. One end of electromagnet 70 is adjacent and in abutting relation to housing 12, and the opposite end, i.e. the left-hand end as viewed in Figure 2, is closed by a plate element 78 fitted within the open end of housing 76 and fitted onto an end of spool 72. Electromagnet 70 is joined to housing 12 in the following manner.

The interior, fluid containing region of housing 12 and the electromagnet 70 are separated by a barrier means of fluid impervious material in the form of a relatively thin plate or diaphragm-like component 80. The end of magnet housing 76 adjacent housing 12 is provided with an annular band 82 around the outer surface and adjacent the axial end face of housing 76. The outer diameter of band 82 when placed on housing 76 is substantially equal to the outer diameter of housing portion 38 so that the respective outer surfaces are substantially flush. The axial end faces of band 82 and magnet housing 76 are coplanar. The housing and electromagnet structures are placed in abutting relation on opposite surface portions of the plate 80, and the assembly secured together by a weld joining the respective adjacent outer surfaces of band 82 and housing portion 38. In addition, an enlarged annular end portion 84 of spool 72 contacts the central portion of plate 80 in a manner supporting the same.

By way of example, in an illustrative pump, spool 72, magnet housing 76 and closure 78 are of ferromagnetic material, for example 0.4750 nickel iron alloy. Plate 80 and band 82 are of titanium, the material of plate 80 being found suitable for use in the exemplary implanted drug delivery pump previously mentioned. Spool 72 has a length of about 14.1 mm (0.555 inch) and a diameter of 2.0 mm (0.079 inch). Housing 76 has a wall thickness of about 0.76 mm (0.03 inch), band 82 a thickness of about 0.51 mm (0.02 inch) and diaphragm 80 a thickness of about 0.025 mm (0.001 inch). Coil 74 has about 3600 turns of 42 gauge wire.

The pump of the present invention further comprises a body 90 of magnetically permeable material in the fluid containing region of housing 12 and between the fluid receiving chamber 14 and the fluid pumping chamber 16. In addition to providing separation between the two chambers, body 90 also defines a portion of the magnetic circuit in the pump, along with other components of the pump, in a manner which will be described. Body 90 is generally solid cylindrical in shape having an outer diameter substantially equal to the diameter of housing inner surface 40 thereby providing a close fitting relationship. Body 90 has a main body portion between axial end surfaces 92 and 94 which is of an axial length less than the distance between housing surface 42 and plate 80 by an amount determined by the desired dimension of pumping chamber 16. Body 90 is formed to include an outer annular rim portion 96 extending from end face 92 and which abuts a corresponding surface portion of plate 80 as shown in Figure 2. The radial thickness of rim portion 96 is substantially equal to that of magnet housing 76, and the two are in substantial alignment for maximizing transmission of magnetic flux therebetween in a manner which will be described. Body 90 is provided with a central through bore or passage 98 of substantial diameter for receiving a portion of the pump armature in a manner which will be described. The main portion of body 90 also is provided with a smaller through bore or passage 100 offset from passage 98 and of substantially the same diameter and in registry with outlet passage 44 thereby providing fluid communication between pumping chamber 16 and the outlet 20. Body 90 is positioned in housing 12 by means of a rod 102 extending through corresponding bores in body 90 and valve housing portion 28 shown in Figure 2, the rod 102 preferably being of Teflon material and the parts being secured together by a compression fit.

By way of example, in an illustrative pump, body 90 is of mu metal which is selected to provide the desired degree of magnetic permeability while at the same time being compatible with medicine or the like for use in the exemplary implanted drug delivery pump previously mentioned. As is well known, mu metal includes nickel in a major portion with the balance including iron, copper and chromium. In addition, some or all of the external surfaces of body 90 can be plated or coated with gold or other suitable material chosen to enhance the compatibility of the drug with the external surface. The outer diameter of body 90 is about 6.6 mm (0.26 inch), the axial length between end face 94 and the end face of rim 96 is about 3.30 mm (0.130 inch), and the axial length between end faces 92, 94 is about 2.5 mm (0.1 inch). Passage 100 has a diameter of about 0.74 mm (0.029 inch) and the central passage 98 a diameter of about 2.5 mm (0.1 inch).

The pump according to the present invention further comprises an armature generally designated 110 positioned in the fluid containing region of housing 12. The armature has a pole portion located for magnetic attraction by the electromagnet 70 and has a plunger portion in the pumping chamber 16 for forcing fluid out of the chamber and outlet 20. The armature 110 is movably supported in housing 12 for movement from a rest position through a forward pumping stroke when attracted by the electromagnet 70 to force fluid out of the pumping chamber 16 through outlet 20, and for movement in an opposite direction through a return stroke back to the rest position. In Figure 2, armature 110 is shown in a position at the end of the forward pumping stroke in response to energization of electromagnet 70, and in Figure 3 armature 110 is shown in the rest position at the end of the return stroke which will be described in detail presently.

Armature 110 includes a shaft or rod portion 112 which is positioned in housing 12 with the longitudinal axis thereof generally coincident with the longitudinal axis of housing 12. A major portion of the length is a section of relatively small diameter. The armature further includes an enlarged body portion 114 of magnetically permeable material which provides the armature pole portion and the plunger portion in a manner which will be described. Body 114 is solid cylindrical in shape having an outer diameter slightly smaller than the diameter of passage 98 in body 90. This is to allow reciprocal movement of armature body 114 within the body 90 during the forward and return strokes of armature 110. In addition, the sizes of the outer diameter of body 114 and the diameter of passage 98 are selected to provide a fluid leakage path from receiving chamber 14 to pumping chamber 16 during the armature return stroke in a manner which will be described. The armature body 114 terminates at the end facing electromagnet 70 in an axial end face 116 which serves as the pole face and is disposed substantially perpendicular to the armature axis. The pole face 116 together with electromagnet 70 define the magnetic circuit gap which is closed during the forward pumping stroke. The pole face 116 is of relatively large cross-sectional area as compared to the cross sectional area of the armature shaft portion 112. The body 114 also serves as the plunger portion of the armature because as the pole face 116 moves toward plate 80 during the forward stroke when magnet 70 is energized, body 114, upon moving into pumping chamber 116 displaces fluid therefrom forcing it out through passages 100, 44 to outlet 20.

Shaft portion 112 is fixed to body 114 in the following manner. Body 114 is provided with a longitudinal bore extending inwardly from the opposite axial end face 118 which terminates within body 114 at a location spaced from pole face 116. A sleeve 120 of fluoropolymer material such as Teflon is fitted in the bore, and the end of the armature shaft portion 112 is fixed in the sleeve or bushing 120. The foregoing is provided by a mechanical compression fit.

Armature 110 includes two relatively larger diameter shaft sections spaced axially from body 114 in a direction opposite therefrom. In particular, there is a first section 124 facing body 114 and a second, axially adjacent section 126 which of slightly larger diameter. The two sections 124, 126 are of relatively short axial length, and they define therebetween an annular shoulder facing the body 114.

There is also provided biasing means in the form of a coil spring 130 for urging armature 110 toward the rest position shown in Figure 3. One end of spring 130 seats in the annular shoulder defined by the armature shaft sections 124, 126. The opposite end of spring 130 seats in an annular spring retainer element 134 which has an annular rim portion which abuts against the end face 94 of body 90 as shown in Figure 2. The annular shape of retainer 134, with the two diameter rim sections, enables it to be located concentric with the armature shaft section 112 to receive the spring 130 which also is concentric with the shaft, while at the same time not interfering with body 114 during movement of the armature 110. The outer diameter of the largest rim portion of retainer 134 is substantially equal to the diameter of surface 32, and retainer 134 is merely located within the housing 12, being held in place by the force of spring 130.

Armature 110 has a valve portion at the axial end of the armature opposite that of the pole face 116 for closing the inlet 18 when the armature is in the rest position and for opening inlet 18 when the armature is moved during the forward pumping stroke. There is provided an armature shaft section 138 having an outer diameter slightly less than the diameter of passage 62 in body 56. Section 138 terminates in a conical tip formation 140 at the axial end of the armature. The tip 140 is adapted for movement into and out of seating engagement with a body 142 fitted in passage 62 and having a longitudinal passage 144 therein in communication with inlet passage 64. In particular, the surface of cone 140 contacts the edge of opening 114 during seating of the valve to block fluid communication between inlet 18 and receiving chamber 14. In the device shown, passage 114 is of smaller diameter than passage 64. The tip 140 is of metal and the element 142 is of relatively soft material, for example silicone rubber, to facilitate the seating of element 140 therein.

By way of example, in an illustrative pump, the armature rod or shaft portion including the sections 112, 124, 126, 138 and 140 is machined from metal, preferably titanium for use in the aforementioned illustrative implanted drug delivery pump. Armature body 114 is of mu metal, retainer 134 is of titanium and body 142 can be cut from a length of silicone rubber tubing. In addition, armature body can be coated or plated with gold, or other suitable material, like body 90, chosen to enhance the compatibility of the drug with the external surface. Also, the outer surface of body 114 received in passage 98 can be coated with Teflon to reduce friction. The armature shaft or rod portion has an overall length of about 8.6 mm (0.34 inch) from the end fitted within body 114 to the end of the conical tip 140. The smaller diameter section 112 of major axial length has a diameter of about 0.91 mm (0.036 inch), and the relatively larger diameter sections 124 and 126 have diameters of about 2.36 mm (0.093 inch) and 3.45 mm (0.136 inch), respectively, and axial lengths of about 0.89 mm (0.035 inch) and 0.38 mm (0.015 inch), respectively. Section 138 has a diameter of about 1.72 mm (0.068 inch) and a length of about 1.47 mm (0.058 inch), and conical tip 140 has an axial length of about 0.94 mm (0.037 inch) and a base angle of about 60°. Body 114 has an overall axial length of about 3.56 mm (0.14 inch) and an outer diameter of about 2.5 mm (0.1 inch). Spring 130 is 0.006 titanium wire and the passage 144 in body 142 has a diameter of about 0.38 mm (0.015 inch).

In operation, inlet 18 is connected to a source or supply of fluid to be pumped, and conduit 24 leading from check valve 22 is connected to a point or location of use for the pumped fluid. The armature 110 is moved through the forward pumping stroke in response to electrical energization of electromagnet 70. One way of energizing magnet 70 is to charge a capacitor from a battery and then discharge that capacitor through coil 74. Other procedures can of course be employed for electrically energizing coil 74 in a known manner. Prior to electrical energization of magnet 70, armature 110 is in the rest position illustrated in Figure 3 where the valve at the end of armature 110 in the form of conical tip 140 is seated in opening 114 to block fluid communication from inlet 18 to the fluid receiving chamber 14. In the rest position of armature 110, pole face 116 is spaced from diaphragm 80 as shown in Figure 3 thereby defining the gap in the magnetic circuit. In the rest position this gap between pole face 116 and diaphragm 80 is of maximum length.

When coil 74 is electrically energized, the armature pole portion 114 is attracted toward magnet 70 thereby causing armature 110 to be drawn toward diaphragm 80. Electromagnetic flux travels through the magnetic circuit including the electromagnet core 72, plate 78, magnet housing 76, rim 96 of body 90, the included portion of diaphragm 80 between housing 76 and rim 96, body 90, armature pole portion 114, and the gap between pole face 116 and diaphragm 80. As armature 110 is moved in the forward pumping stroke, i.e. in a direction to the left as viewed in Figures 2 and 3, fluid initially contained in the pumping chamber 16, i.e. the region between body 90, diaphragm 80 and pole portion 114, is thereby forced through passages 100 and 44 out through the outlet 20 and through check valve 22 to the conduit 24. The clearance between armature pole portion 114 and the passage 98 in body 90 is selected to be sufficiently small so that fluid leakage therebetween is relatively small during the forward pumping stroke of armature 110. During the forward pumping stroke, the armature valve portion 140 becomes unseated from the opening of passage 144 thereby allowing fluid flow from inlet 18 into receiving chamber 14 thereby filling the same. The forward pumping stroke of armature 110 is completed when pole face 116 approaches contact with diaphragm 80. Actual contact may not be achieved since viscosity limits outflow of the fluid between the pole face 116 and diaphragm 80.

When electrical excitation of coil 74 ceases, armature 110 is moved in the opposite direction, i.e. to the right as viewed in Figures 2 and 3, by the force of biasing spring 130 until the armature reaches the rest position as shown in Figure 3 with conical tip 140 seated in the opening of passage 144. This motion is relatively slow since it is limited by the small leak rate of fluid between the outer surface of pole portion 114 and the surface of passage 98 in body 90 at a pressure difference determined by the force applied by the spring 130. Thus fluid leakage fills chamber 16 from chamber 14. Thus, the average pumping rate which is determined by the rate of return of armature 110 to the rest position can be relatively slow, but such a pumping rate is called for in typical implantable drug delivery systems. Armature 110 then remains in the rest position of Figure 3 closing inlet 18 and waiting for the next forward pumping stroke which occurs when magnet 70 is energized again.

The non-movable diaphragm 80 of titanium or like material provides an hermetic seal between the fluid in housing 12 and the electrical components associated with magnet 70. Having armature 110 immersed in the fluid makes operation of the pump nearly independent of ambient pressure. The initial condition of the pump when armature 110 is in the rest position of Figure 3 is that the fluid is at substantially the same pressure on opposite sides of the pole portion 114, i.e. in the receiving chamber 14 and in the pumping chamber 16.

The pump of the present invention is made electrically and magnetically efficient by minimizing the total gap within the magnetic circuit, by having the magnetic pole face 116 of relatively large surface area, and by having core 72 of relatively small diameter. In particular, there is a relatively large contact area at the interface between the axial end face of magnet housing 76 and diaphragm 80 and between diaphragm 80

**0 102 824**

and the axial end face of rim 96 of body 90 to minimize the effective air gap introduced by diaphragm 80 at this point in the magnetic circuit. Related to this is the need for welding diaphragm 80 to the band 82 and housing port 38 to achieve a hermetic seal between electromagnet 70 and the fluid containing region of housing 12 while at the same time not adversely affecting the magnetic circuit. In addition, there is a relatively large surface area along the gap between body 90 and pole portion 114 to minimize the effective air gap introduced at this point in the magnetic circuit. The relatively small diameter of core 72 provides the necessary ampere turns with a minimum electrical resistance. The large area of pole face 116 provides a high magnetic force with a minimum number of ampere turns. Having the magnetic gap external to coil 74, i.e. between pole face 116 and diaphragm 80, allows the foregoing features to be achieved simultaneously. The external Teflon coating over the gold plating on pole portion 114 reduces magnetic side loading on armature pole portion 114 and reduces operating friction. The combination of the conical formation 140 and the relatively soft body 142 enables the armature 110 to be self-aligning and permits relatively loose tolerance in construction. The pump of the present invention is relatively small in size having an overall diameter of around 7 millimeters and an overall length of about 27 millimeters, and has the relatively light weight of about 4.5 grams.

Figures 8—14 show a pump according to another embodiment of the present invention. In this embodiment the pump includes a check valve which is integral with the armature, i.e. the check valve is operatively coupled to the armature and is located in the fluid-receiving region of the housing for opening and closing the pump inlet. In particular, in this embodiment of the present invention the check valve comprises a valve member movably carried by the armature and positioned for closing the pump inlet when the armature is in the rest position and for opening the inlet after the armature begins movement associated with the forward pumping stroke. For convenience in illustration, components of the pump of this embodiment which are similar to those of the pump of Figures 1—7 are identified by the same reference numerals provided with a prime designation.

The pump of this embodiment has an armature generally designated 150 including a shaft section 152 of relatively small diameter adjacent one end of the armature, i.e. the left-hand end as viewed in Figure 8. Shaft section 152 is similar to section 112 in the embodiment of Figures 1—7 but is of relatively shorter axial length. An armature pole portion 114' having a pole face 116' and identical to the pole portion and pole face of the armature of Figures 1—7 is fixed to this end of shaft section 152 by a compression fit between pole portion 114', a Teflon busing 120' and the shaft section in a manner identical to that of the preceding embodiment. Armature 150 includes a shaft section 154 of slightly larger diameter adjacent shaft section 152 in a direction away from end face 118' of pole portion 114'. Section 154, in turn, meets an armature body portion 158 of larger diameter and formed to include a plurality, in the present illustration four, radially extending formations or lands 160a—160d as shown also in Figure 12. Each land formation 160a—160d has a relatively short, curved outer edge 162a—162d, respectively, close to the inner surface 32' for guiding the armature 150 as it moves within the housing. Each land is separated from an adjacent land by a corresponding straight edge 163, 165 and 166 as shown in Figure 12 thereby providing a flow space of significant area between the particular straight edge and the corresponding curved portion of surface 32'. The lands 160a—160d also define a plurality of shoulders facing in opposite axial directions for a purpose to be described. Armature 150 terminates in an axial end face 167 disposed toward inlet 18'. A longitudinal bore 168 extends inwardly from face 167 along within body 158 and terminates in the shaft section 154 for movably carrying the check valve member 170 which now will be described.

Check valve 170 has a main body 172 and an axial face 174, and the check valve 170 is positioned with face 174 disposed toward the armature end face 167. Check valve 170 has a rod or shaft portion 178 extending outwardly in a direction normal to end face 174 and is received in armature passage 168 in slidably movable relation. The armature shaft portion 154 is provided with a pair of oppositely directed radially extending bores or passages 182 in communication with the longitudinal passage 168 at the end where check valve shaft portion 178 terminates for a purpose to be described. The check valve body 172 has a relatively large diameter portion 184 of relatively short axial length defining an annular shoulder facing toward the lands 160a—160d of the armature. The opposite axial end of valve member 170 includes a frustoconical surface 186, a cylindrical surface 188 and a conical top formation 190. These portions of valve 170 extend into an annular valve seat element 192 having a central bore or passage 194, which, in turn, is fixed in the annular recess of body 56' of the fitting defining inlet 18'.

There is also provided first biasing means in the form of coil spring 200 between a spring retainer 134' and the annular shoulders defined by lands 160a—160d for urging armature 150 into the rest position. There is also provided second biasing means in the form of coil spring 204 positioned between the annular shoulder defined by lands 160a—160d and the annular shoulder defined by valve section 184 for urging check valve 170 into the position closing inlet 18'.

By way of example, in an illustrative pump, the components identified by the primed reference numerals are of the same material and of substantially the same dimensions as corresponding components in the embodiment of Figures 1—7. With respect to armature 160, pole portion 114' is the same material and of substantially the same size as pole portion 114 in Figures 1—7. The remainder of the armature 150 is of titanium, and the overall length measured between the left-hand end of the shaft portion 152 as viewed in Figure 8 to axial end face 167 is about 7.24 mm (0.285 inch). Shaft section 152 has a length of about 3.30 mm (0.130 inch) and a diameter of about 0.914 mm (0.036 inch). Shaft section 154 has a length

7

of about 2.03 mm (0.080 inch) and a diameter of about 1.40 mm (0.055 inch). Body 158 has a length of about 1.90 mm (0.075 inch) and a diameter of about 2.36 mm (0.093 inch). Each of the land portions 160a—160d has an axial length of about 1.02 mm (0.040 inch), each edge 162a—162d has a length of about 0.51 mm (0.020 inch), and edges 162a—162d lie about on a circle having a diameter of about 3.71 mm (0.146 inch).

Valve member 170 is of titanium, and has an overall length of about 5.46 mm (0.215 inch) between the outer end face of shaft portion 178 to the top of core 190. Shaft portion 178 has a length of about 2.54 mm (0.100 inch) and a diameter of about 0.635 mm (0.025 inch). The two sections of body 172 have outer diameters of 2.36 mm (0.093 inch) and 3.45 mm (0.136 inch). Surface 186 has a base angle of about 60° and an axial length of about 0.76 mm (0.03 inch). The combined axial lengths of surfaces 188 and 190 is about 1.27 mm (0.050 inch), surface 188 has a diameter of about 0.762 mm (0.030 inch) and surface 190 has a base angle of about 60°. Valve seat member 192 is of silicone rubber tubing having an outer diameter of about 2.54 mm (0.100 inch) and an inner diameter of about 1.02 mm (0.040 inch). Spring 200 provides a force of about 3.5 grams and spring 204 a force of about 1.5 grams.

In operation, inlet 18' is connected to a source or supply of fluid to be pumped, and outlet 20' is connected to a point or location of use for the pumped fluid. The armature 150 is moved through the forward pumping stroke in response to electrical energization of electromagnet 70'. As in the proceeding embodiment, one way of energizing magnet 70' is to charge a capacitor from a battery and then discharge that capacitor through coil 74. Other procedures can of course be employed for electrically energizing coil 74' in a known manner. Prior to electrical energization of magnet 70', armature 150 is in the rest position illustrated in Figure 9 where the check valve 170 is located with frustoconical surface 186 seated in the opening of passage 194 to block fluid communication from inlet 18' to the fluid receiving chamber 14'. In the rest position of armature 150, pole face 116' is spaced from diaphragm 80' as shown in Figure 9 thereby defining the gap in the magnetic circuit. In the rest position this gap between pole face 116' and diaphragm 80' is of maximum length.

When coil 74' is electrically energized, the armature pole portion 114' is attracted toward magnet 70' thereby causing armature 150 to be drawn toward diaphragm 80. Electromagnetic flux travels through the magnetic circuit including the electromagnet core 72' plate 78', magnet housing 76', rim 96' of body 90', the included portion of diaphragm 80 between housing 76' and rim 96', body 90', armature pole portion 114', and the gap between pole face 116' and diaphragm 80'. As armature 150 is moved in the forward pumping stroke, i.e. in a direction to the left as viewed in Figures 8—10, fluid initially contained in the pumping chamber 16', i.e. the region between body 90', diaphragm 80' and pole portion 114', is thereby forced through passages 110' and 44' and out through the outlet 20'. The clearance between armature pole portion 114' and the passage 98' in body 90' is selected to be sufficiently small so that fluid leakage therebetween is relatively small during the forward pumping stroke of armature 150.

The check valve member 170 slides freely with respect to the armature 150 and does not necessarily move when the armature 150 is drawn toward diaphragm 80'. Such relative positions are illustrated, for example, in Figure 10. At rest, the surface 186 of valve member 170 is held in contact with the valve seat defined by passage 194 by the spring 200 acting upon the armature 150 which is then in contact with the check valve member 170 as shown in Figure 9. When the armature 150 is drawn toward diaphragm 80' the force of spring 200 is no longer transferred to the check valve member 170 and the force holding the valve member 170 against the valve seat is decreased to that provided by spring 204, which generally provides a force less than that provided by spring 200. If armature 150 is drawn toward electromagnet 70' with sufficient velocity, pressure within the pump housing 12' between body 90' and the check valve seat decreases to a level below the level at the pump inlet 18' and the net force due to fluid pressure from inlet 18' acting on the check valve member 170 tends to move the valve member 170 away from its seat. If the net force due to the fluid pressure exceeds that applied by the spring 200, then valve member 170 moves away from the valve seat and fluid flows into the pump body as illustrated in Figure 8. In fact, because the fluid is nearly incompressible the check valve 170 opens at approximately the same time that the armature 150 achieves enough velocity to force fluid out of the pump outlet 20'. The forward pumping stroke of the armature 150 is completed when the pole face 116' approaches contact with the diaphragm 80'. Actual contact may not be achieved since viscosity limits outflow of the fluid between the pole face 116' and the diaphragm 80. When the armature velocity decreases to a level such that the displacement rate of the motion of the pole portion 114' no longer exceeds the leak rate between the pole portion 114' and the body 90', the pressure within the pump housing 12' begins to increase. When the force due to the pressure difference across the check valve member 170 no longer exceeds the force of spring 204, the check valve member moves toward the valve seat and prevents flow out of the inlet port 18' of the pump as illustrated, for example, in Figure 10.

When electrical excitation of coil 74' ceases, armature 150 is moved in the opposite direction, i.e. to the right as viewed in Figures 8—10, by the force of biasing spring 200 until the armature reaches the rest position as shown in Figure 9 with surface 186 seated in the opening of passage 194. This motion is relatively slow since it is limited by the small leak rate of fluid between the outer surface of the pole portion 114' and the surface passage 98' in body 90' at a pressure difference determined by the force applied by the spring 200. During the return stroke of armature 150 the check valve member 170 is held against the valve seat primarily by the light spring 204 supplemented by the difference between the outlet and inlet pressures acting on the valve seat. Openings 182 allow exit of fluid from the space between passage 168

8

and valve portion 178. When the return stroke has been completed the spring force is increased to that of spring 200. Thus, the average pumping rate which is determined by the rate of return of armature 150 to the rest position can be relatively slow, but such a pumping rate is called for in typical implantable drug delivery systems. Armature 150 then remains in the rest position of Figure 9 with inlet 18' closed and waiting for the next forward pumping stroke which occurs when magnet 70' is energized again.

The pump of the embodiment of Figures 8—14 has all the advantages of the embodiment of Figures 1—7 including the diaphragm 80' providing an hermetic seal between the fluid in housing 12' and the electrical components associated with magnet 70', the immersed armature 150 making operation of the pump nearly independent of ambient pressure, the electric and magnetic efficiency being provided by minimizing the total gap within the magnetic circuit, by having the magnetic pole face 116' of relatively large surface area, and by having core 72' of relatively small diameter, and the pump being relatively small in size and light in weight. In addition, the pump of this embodiment has the further advantage of an integral check valve.

The pump of this embodiment of the present invention is illustrated further by the following example and data. A pump as shown in Figures 8—14 was pulsed by a 4.7 microfarad capacitor at 15 volts to pass 0.31 microliters against a 0.29 kPa (2 p.s.i.) head. At a 1 milliliter per day flow rate (3226 pulses per day) the calculated five year energy requirement from the capacitor is 3100 joules. This is approximately 12% of the energy available from a model 7911 lithium-iodine cell commercially available from Wilson Greatbatch Ltd., Clarence, New York.

Tables I and II present performance data obtained from operating a pump as shown in Figures 8—14 with water, a coil resistance of 243 ohms, a 4.7 microfarad capacitor, and with capacitor charging voltages of 15 volts and 20 volts, respectively:

TABLE I

Volume delivered per pulse vs. pressure increase
capacitor charging voltage of 15 volts

| Pressure increase across pumps in kPa (p.s.i.) | Volume delivered per pulse in microliters |
|---|---|
| 0     (0) | 0.40 |
| 0.29  (2.0) | 0.31 |
| 0.51  (3.5) | 0.20 |
| 0.725 (5.0) | 0.10 |

TABLE II

Volume delivered per pulse vs. pressure increase
capacitor charging voltage of 20 volts

| Pressure increase across pumps in kPa (p.s.i.) | Volume delivered per pulse in microliters |
|---|---|
| 0     (0) | 0.40 |
| 0.29  (2.0) | 0.35 |
| 0.51  (3.5) | 0.25 |
| 0.725 (5.0) | 0.20 |

Values for volume delivered per pulse and pressure increase across the pump were substantially the same using a capacitor charging voltage of 25 volts.

Tables III and IV present performance data for the pump with water, coil resistance of 243 ohms, 0.29 kPa (2 p.s.i.) pressure difference across the pump, and capacitor magnitudes of 3.3 and 4.7 microfarads, respectively:

TABLE III

Capacitor energy per milliliter vs. capacitor
voltage; capacitance of 3.3 microfarads

| Capacitor charging voltage in volts | Energy per milliliter delivered in joules per milliliter |
|---|---|
| 15.0 | 1.3 |
| 20.0 | 2.2 |
| 25.0 | 3.1 |

TABLE IV
Capacitor energy per milliliter vs. capacitor
voltage; capacitance of 4.7 microfarads

| Capacitor charging voltage in volts | Energy per milliliter delivered in joules per milliliter |
|---|---|
| 10.0 | 1.1 |
| 15.0 | 1.7 |
| 20.0 | 2.8 |
| 25.0 | 4.2 |

In the pump from which the foregoing data was obtained, the forward pumping stroke has a duration from about 1 to about 5 milliseconds, dependent upon the voltage and the back pressure, and the return stroke has a duration from about 0.1 to about 1.5 seconds.

Figures 15—18 illustrate a modified form of check valve 210 in the pump of Figures 8—14. The check valve 210 of Figures 15—18 includes a main body portion 212 similar to body 172 of valve member 170 in Figures 8—14 and a shaft portion 214 movably received in a longitudinal face of the armature. The body 212 is formed to include a plurality of lands 216a—216d similar to lands 160a—160d which define shoulders for engaging the end of spring 204. The end face of body 212 facing inlet 18' is provided with a recess to accommodate a disc-shaped valve element 220 and a spring-member 224. Disc 220 is of silicon rubber and spring member 224 is a thin disc of titanium foil contoured as shown in Figures 15 and 16 to enable disc 220 to rock within the recess. Thus disc 220 is captive within the recess but not attached to it. The passage 64' of the fitting providing inlet 18' terminates in an outwardly flared surface portion 228 defining a valve seat edge 130 of diameter layer than passage 64'. Disc 220 is of slightly larger diameter and is adapted to seat on edge 226. Figures 15 and 16 illustrate the unseated and seated conditions of the check valve. The ability of disc 220 to rock within the recess by virtue of spring 224 ensures that disc 220 lies flat on valve seat 230.

The check valve of Figures 15—18 has a number of advantages. The flow area in the open portion at the same stroke is relatively larger, and therefore the pressure drop across the check valve is relatively lower. There is no possible leak path around the back of the seat. Seating of valve element 220 on seat 230 does not involve a rubbing motion, and therefore the possibility of unacceptable wear on the seat is reduced. In addition, the arrangement of Figures 15—18 is relatively easy to manufacture.

By way of example, in an illustrative pump, valve element 220 has a diameter of about 2.54 mm (0.100 inch) and a thickness of about 0.762 mm (0.030 inch), spring 224 has a diameter of about 2.54 mm (0.100 inch) and a thickness of about 0.051 mm (0.002 inch), and surface 228 defines an angle of about 45° with the housing longitudinal axis.

Referring now to Figures 19—22 a pump 310 according to another embodiment of the present invention includes a housing 312 which is generally hollow cylindrical in overall shape and includes an interior region for containing fluid, i.e. the liquid to be pumped. As shown in Figure 20, the hollow interior region is divided in a manner which will be described into a fluid receiving chamber 314, a first fluid pumping chamber 316 in fluid communication with receiving chamber 314, and a second fluid pumping chamber 318 serially in fluid communication with pumping chamber 316. There is an inlet generally designated 320 in fluid communication with the receiving chamber 314 and adapted to be connected to a source or supply of fluid to be pumped. There is also an outlet 322 in fluid communication with the second pumping chamber 318 and adapted to be in fluid communication with a location to which the fluid is to be pumped. There is provided check valve means operatively associated with the fluid containing region of pump 310 for allowing fluid flow in a direction from the inlet 320 through outlet 322 and blocking fluid flow in a direction from the outlet through the inlet. In the pump shown the check valve is within the pump and associated with the pump armature in a manner which will be described.

Referring now to Figure 20, housing 312 is generally hollow cylindrical in overall shape including a main body portion 326 of relatively substantial wall thickness. Housing portion 326 has a first axial end section 328 extending from one end of housing portion 326, i.e. the right-hand end as viewed in Figure 20, which is of relatively smaller wall thickness and terminates in an axial end face 330. Portion 326 and section 328 define an interior surface of constant diameter. Thus, the outer surface of housing portion 326 and section 328 are of different diameters and meet in an annular surface 334. The housing portion 326 has a second axial end section 338 extending from the other end of housing section 326, i.e. the left-hand end as viewed in Figure 20, which also is of smaller wall thickness. End section 338 has a relatively larger diameter inner wall surface 340 which meets surface 332 in annular intermediate wall 342.

Main body portion 326 is provided with a longitudinal bore or passage 344 for placing the second pumping chamber 318 in fluid communication with outlet 322. Outlet 322 is located on the side of housing 312 for communication with the passage 344. Housing portion 328 is provided with a radially extending opening in the outer wall thereof in communication with passage 344.

A housing extension portion 346 is provided between main body portion 328 and inlet 320. Portion 346 is generally cylindrical having first and second axial sections 348 and 350, respectively. Section 348 is received snugly within housing section 328 with section 348 having an outer diameter substantially the same as the diameter of surface 332. Section 348 has an inner surface 352 and terminates in an axial end face 354 exposed to the first pumping chamber 316. Section 350 has an outer diameter substantially equal to the outer diameter of housing section 328 whereby the outer surfaces thereof are substantially flush. The outer surface of section 350 meets the smaller diameter outer surface of section 348 in an annular surface 356 which abuts end face 330. Section 350 has an inner surface 358 of a diameter larger than that of inner surface 352 of section 348, and the two inner surfaces 352, 358 meet in an annular surface 360. Section 350 terminates in an axial end face 362.

Inlet 320 is provided by a plug-like element which is fitted into the open end of section 350 of the housing extension portion 346. The element includes a main body 336 generally cylindrical in shape and having an outer diameter substantially equal to the diameter of inner surface 358 of section 350 thereby providing a close fit therein. Body 366 has one axial end face 368 located in chamber 314, and adjacent the opposite end face the outer surface of body 366 is provided with an annular rim 370 which abuts the annular end face 362 for placement and securement of the fitting in the housing. The two components are secured together by welding or other suitable means. An internal bore 372 extends between the two opposite axial end faces of body 366 to provide an internal passage for the inlet. Passage 372 meets the inner axial end face 368 an annular, conical shaped surface 374. Inlet 320 is adapted for connection to a conduit such as a flexible tubing leading from a source or supply of fluid to be pumped.

By way of example, in an illustrative pump, housing 312 including main body portion 326 and extension portion 346, together with the inlet and outlet fittings 318 and 320, respectively, all are of metal, and for a drug delivery pump for implantation in a patient, titanium has been found to provide satisfactory results. In such an illustrative pump, housing 312 has an overall length of about 14.1 mm (0.555 inch) measured between the axial end faces of section 338 and 350. Surface 332 has a diameter of about 3.81 mm (0.15 inch), and the axial end face 330 has a radial dimension of about 0.762 mm (0.03 inch). Surface 340 has a diameter of about 6.6 mm (0.26 inch), and the axial end face of housing section 338 has a radial thickness of about 0.51 mm (0.02 inch). Passage 334 in housing body 326 and the interior passage in outlet fitting 322 both have a diameter of about 0.813 mm (0.032 inch). Surface 352 has a diameter of about 2.54 mm (0.100 inch), and axial end face 354 has a radial dimension of about 1.27 mm (0.050 inch). Surface 358 has a diameter of about 3.43 mm (0.135 inch), and axial end face 362 has a radial dimension of about 1.02 mm (0.040 inch). The axial distance between end faces 354 and 362 is about 6.60 mm (0.260 inch). In the inlet plug fitting 320, the passage 372 has a diameter of about 0.86 mm (0.034 inch) and surface 374 is disposed at an angle of about 45 degrees to the longitudinal axis of fittings 320.

The pump of the present invention further comprises electromagnet means generally designated 380 carried by housing 312 and located external to the fluid containing region of the housing. As shown in Figure 20, the electromagnet 380 includes a core 382 in the form of a spool which is generally solid cylindrical in shape. A coil 384 is wound on spool 382 and contained within a hollow housing 386 generally cylindrical in shape. One end of electromagnet 380 is adjacent and in abutting relation to housing 321, and the opposite end, i.e. the left-hand end as viewed in Figure 20, is closed by a plate element 388 fitted within the open end of housing 386 and fitted onto an end of spool 382. Electromagnet is joined to housing 321 in the following manner.

The interior, fluid containing region of housing 321 and the electromagnet 380 are separated by a barrier means of fluid impervious material in the form of a relatively thin plate or diaphragm-like component 390. The end of magnet housing 386 adjacent housing 312 is provided with an annular band 392 around the outer surface and adjacent the axial end face of housing 386. The outer diameter of band 392 when placed on housing 386 is substantially equal to the outer diameter of housing section 338 so that the respective outer surfaces are substantially flush. The axial end faces of band 392 and magnet housing 386 are co-planar. The housing and electromagnet structures are placed in abutting relation on opposite surface portions of the plate 390, and the assembly secured together by a weld joining the respective adjacent outer surfaces of band 392 and housing section 338. In addition, an enlarged annular end portion 394 of spool 382 contacts the central portion of plate 390 in a manner supporting the same.

By way of example, in an illustrative pump, spool 382, magnet housing 386 and closure 388 are of ferromagnetic material, for example 4750 nickel iron alloy. Plate 390 and band 392 are of titanium, the material of plate 390 being found suitable for use in the exemplary implanted drug delivery pump previously mentioned. Spool 382 has a length of about 14.1 mm (0.555 inch) and a diameter of 2.01 mm (0.079 inch). Housing 386 has a wall thickness of about 0.76 mm (0.03 inch), band 392 a thickness of about 0.51 mm (0.02 inch) and diaphragm 390 a thickness of about 0.025 mm (0.001 inch). Coil 384 has about 3600 turns of 42 gauge wire.

The pump of the present invention further comprises a body 400 of magnetically permeable material in the fluid containing region of housing 312 and between the first and second fluid pumping chambers 316 and 318, respectively. In addition to providing separation between the two chambers, body 400 also defines a portion of the magnetic circuit in the pump, along with other components of the pump, in a manner which will be described. Body 400 is generally solid cylindrical in shape having an outer diameter substantially equal to the diameter of housing inner surface 340 of housing section 338 thereby providing a close fitting relationship. Body 400 has a main body portion between axial end surfaces 402 and 404 which is of an axial length less than the distance between housing surface 342 and plate 390 by an amount determined by the desired dimension of pumping chamber 318. Body 400 is formed to include an outer annular rim portion 406 extending from end face 402 and which abuts a corresponding surface portion of plate 390 as shown in Figure 20. The radial thickness of rim portion 406 is substantially equal to that of magnet housing 386, and the two are in substantial alignment for maximizing transmission of magnetic flux therebetween in a manner which will be described. Body 400 is provided with a central through bore or passage 408 of substantial diameter for receiving a portion of the pump armature in a manner which will be described. The main portion of body 400 also is provided with a smaller through bore or passage 410 offset from passage 408 and of substantially the same diameter and in registry with outlet passage 344 thereby providing fluid communication between pumping chamber 318 and the outlet 322. Body 400 is positioned in housing 312 by means of a rod 412 extending through corresponding bores in body 400 and valve housing portion 326 shown in Figure 20, the rod 412 preferably being of Teflon material and the parts being secured together by a compression fit.

By way of example, in an illustrative pump, body 400 is of mu metal which is selected to provide the desired degree of magnetic permeability while at the same time being compatible with medicine or the like for use in the exemplary implanted drug delivery pump previously mentioned. As is well known, mu metal includes nickel in a major portion with the balance including iron, copper and chromium. In addition, some or all of the external surfaces of body 400 can be plated or coated by palladium or other suitable material selected to enhance the compatibility of the drug with the external surface. The outer diameter of body 400 is about 6.6 mm (0.26 inch), the axial length between end face 404 and the end face of rim 406 is about 3.31 mm (0.130 inch), and the axial length between end faces 402, 202 is about 2.5 mm (0.1 inch). Passage 410 has a diameter of about 0.74 mm (0.029 inch) and the central passage 408 a diameter of about 2.5 mm (0.1 inch).

The pump according to the present invention further comprises an armature generally designated 420 positioned in the fluid containing region of housing 312. The armature has a pole portion located for magnetic attraction by the electromagnet 380, a first plunger portion associated with the first pumping chamber 316 for forcing fluid from chamber 316 into chamber 318, and a second plunger portion associated with the second pumping chamber 318 for forcing fluid out of chamber 318 and outlet 322. The armature 420 is movably supported in housing 312 for movement from a rest position through a forward pumping stroke when attracted by the electromagnet 390 to force fluid out of the pumping chambers 316 and 318 through outlet 322, and for movement in an opposite direction through a return stroke back to the rest position. In Figure 20, armature 420 is shown in a position at the end of the forward pumping stroke in response to energization of electromagnet 380, in Figure 21 armature 420 is shown in the rest position at the end of the return stroke, and in Figure 22 armature 420 is shown in an energized position and the check valve in a closed position which will be described in detail presently.

Armature 420 includes a shaft or rod portion 422 which is positioned in housing 312 with the longitudinal axis thereof generally coincident with the longitudinal axis of housing 312. Shaft portion 422 is of relatively small diameter. The armature further includes an enlarged body portion 424 of magnetically permeable material which provides the armature pole portion and the second plunger portion in a manner which will be described. Body 424 is solid cylindrical in shape having an outer diameter slightly smaller than the diameter of passage 408 in body 400. This is to allow reciprocal movement of armature body 424 within the body 400 during the forward and return strokes of armature 420. In addition, the sizes of the outer diameter of body 424 and the diameter of passage 408 are selected to provide a fluid leakage path from pumping chamber 316 to pumping chamber 318 during the armature return stroke in a manner which will be described. The armature body 124 terminates at the end facing electromagnet 380 in an axial end face 426 which serves as the pole face and is disposed substantially perpendicular to the armature axis. The pole face 426 together with electromagnet 380 define the magnetic circuit gap which is closed during the forward pumping stroke. The pole face 426 is of relatively large cross-sectional area as compared to the cross sectional area of the armature shaft portion 422. The body 424 also serves as one of the armature plunger portions because as the pole face 426 moves toward plate 390 during the forward stroke when magnet 386 is energized, body 424 upon moving into pumping chamber 318, displaces fluid therefrom forcing it out through passages 400, 344 to outlet 322.

Shaft portion 422 is fixed to body 424 in the following manner. Body 424 is provided with a longitudinal bore extending inwardly from the opposite axial end face 428 which terminates within body 424 at a location spaced from pole face 426. A sleeve 430 or a suitable split bushing of fluoropolymer material such as Teflon is fitted in the bore, and the end of the armature shaft portion 422 is fixed in the sleeve or bushing 430. The foregoing is provided by a mechanical compression fit. In addition, the outer surface of body 424 is provided with a coating 432 of Teflon or like material for a purpose to be described.

Armature 420 includes another body portion 434 spaced axially from body 424, preferably integral with shaft portion 422, and of relatively larger diameter. Body portion 434 provides the armature first plunger portion in a manner which will be described. Body portion 434 has an outer surface extending along a major portion of the axial length of body 434 and having an outer diameter slightly less than the diameter of inner surface 352 of housing section 348. This is to allow reciprocal movement of body portion 434 within housing section 348 during the forward and return strokes of armature 420. In addition, the sizes of the outer diameter of surface 436 and the inner diameter of surface 352 are selected to provide a fluid

12

leakage path from receiving chamber 314 to pumping chamber 316 during the armature return stroke in a manner which will be described.

Body 434 terminates at the end facing body 424 in an annular end face 438 disposes substantially perpendicular to the armature axis and extending between the outer surface of body 424 and the shaft portion 422. Body 434 is formed to include an annular extension or flange 440 between surface 436 and end face 438, located a short axial distance from end face 438. Flange 440 includes opposite side faces 442, 444 and an outer peripheral face 446 spaced from housing inner surface 332. An annular outer surface 448 of body 434 and having a diameter slightly less than that of surface 436 is between flange 440 and end face 438. Surface 448 together with flange face 442 define an annular shoulder facing in an axial direction toward body 424 for a purpose to be described.

There is also provided first biasing means in the form of a coil spring 450 for urging armature 420 toward the rest position shown in Figure 21. One end of spring 450 seats in the annular shoulder described above, and defined by the flange 440 and body surface 448. The opposite end of spring 450 seats in an annular spring retainer element 452 which has an annular rim portion which abuts against the end face 404 of body 400 as shown in Figure 20. The annular shape of retainer 452, with the two diameter rim sections, enables it to be located concentric with the armature shaft section 422 to receive the spring 450 which also is concentric with the shaft, while at the same time not interfering with body 424 during movement of the armature 420. The outer diameter of the largest rim portion of retainer 452 is substantially equal to the diameter of surface 332, and retainer 452 is merely located with the housing 312, being held in place by the force of spring 450.

Body 434 terminates at the end facing inlet 320 in an annular end face 458 disposes substantially perpendicular to the armature axis and meeting an axial extension 460 of relatively short axial length disposed toward inlet 320 and having a diameter approximately equal to the diameter of armature shaft portion 422. Extension 460 operatively engages the pump check valve in a manner which will be described. End face 458 terminates at the outer periphery thereof in an annular surface 462 of body 434 which has a diameter less than the diameter of surface 436 and which extends a relatively small distance axially inwardly from end face 458. Surface 462 terminates in an annular surface 464 disposed substantially perpendicular to the armature axis and which meets surface 436. Surfaces 462 and 464 define an annular shoulder facing inlet 320 for a purpose to be described.

By way of example, in an illustrative pump, the armature 420 including shaft portion 422 and body portion 434 is machined from metal, preferably titanium for use in the aforementioned illustrative implanted drug delivery pump. Armature body 424 is of mu metal and retainer 452 is of titanium. In addition, the armature can be coated or plated with gold, or other suitable material, like body 400, chose to enhance the compatibility of the drug with the external surface. Also, the Teflon coating 432 on the outer surface of body 424 received in passage 408 serves to reduce friction. The combined of armature shaft portion 422 and body portion has an overall length of about 10.4 mm (0.410 inch) from the end fitted within body 424 to the outer end of the extension 460. The armature shaft portion 422 has a diameter of about 0.91 mm (0.036 inch), surface 436 has a diameter of about 2.54 mm (0.100 inch), surface 436 of flange 440 has a diameter of about 3.45 mm (0.136 inch), surface 448 has a diameter of about 2.39 mm (0.094 inch), extension 460 has a diameter of about 0.76 mm (0.030 inch) and surface 462 has a diameter of about 2.03 mm (0.080 inch). From the outer end of extension 460, surfaces 458, 464 and 444 are spaced axially therefrom distances of about 1.02 mm (0.040 inch), 15.2 mm (0.060 inch) and 4.57 mm (0.180 inch), respectively. Flange surface 464 has an axial width of about 0.38 mm (0.015 inch) and the distance between end face 438 and flange surface 444 is about 0.89 mm (0.035 inch). Body 424 has an overall axial length of about 3.6 mm (0.14 inch) and an outer diameter of about 2.5 mm (0.1 inch). Spring 450 is 0.005 titanium wire with the spring inner diameter being about 2.4 mm (0.095 inch).

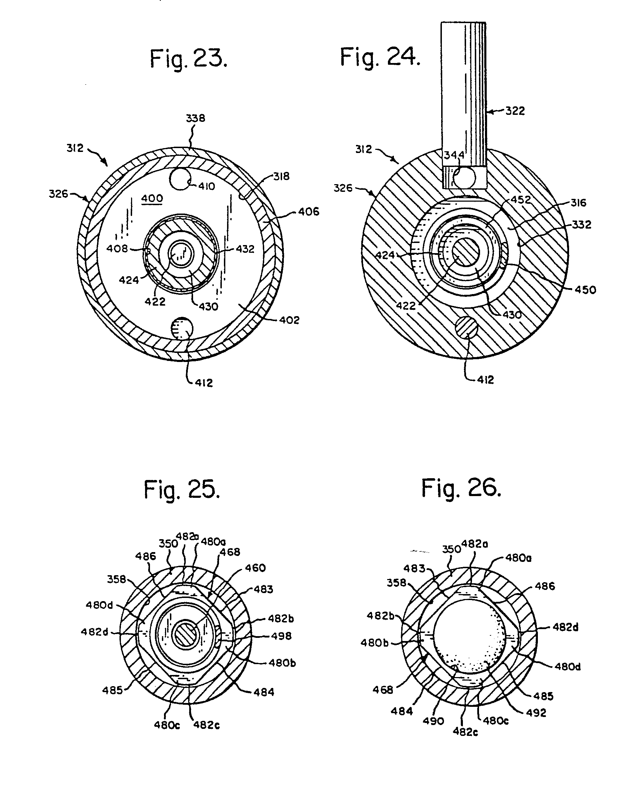

The pump according to the present invention further includes a check valve which is integral with the armature, i.e. the check valve is operatively coupled to the armature and is located in the fluid-receiving region of the housing for opening and closing the pump inlet. In particular, the check valve comprises a valve member movably carried by the armature and positioned for closing the pump inlet when the armature is in the rest position and for opening the inlet after the armature begins movement associated with the forward pumping stroke. As shown in Figures 20—22 the check valve, generally designated 468, is located in fluid receiving chamber 314 between inlet 320 and the end of armature 420 and includes a main body portion 470 generally cylindrical in shape having an outer surface 472 spaced radially inwardly from housing surfaces 352, 358. Surface 472 has a diameter substantially the same as that of surface 462 of body 434. Body portion 470 terminates at the end facing armature 420 in an axial end face 474 disposed substantially perpendicular to the longitudinal axis of armature 420. An axially inwardly extending recess in the form of bore 476 is provided in surface 474 coaxial with the longitudinal axis of armature 420 and extending along within check valve body 470. Recess 476 receives extension 460 of armature body 434. The check valve body 470 is formed to include a plurality, in the present illustration four, radially extending formations or lands 480a—480d as shown also in Figures 25 and 26. Each land formation 480a—480d has a relatively short, curved outer edge 482a—482d, respectively, close to the inner surface 358 of housing section 350 for guiding the check valve 468 as chamber 314. Each land is separated from an adjacent land by a corresponding straight edge 483, 484, 485, and 486 as shown in Figures 25 and 26 thereby providing a flow space of significant area between the particular straight edge and the corresponding curved portion of surface 358. The lands 480a—480d also define a plurality of shoulders facing in an axial direction toward armature 420 for a purpose to be described.

The end face of body 470 facing inlet 320 is provided with a recess 490 to receive a disc-shaped valve element 492 having a smooth, convex outer surface 494 facing inlet 320. In particular, surfaces 374 and 368 of inlet fitting 320 meet in a sharp, annular valve seat edge 196 which is contacted in seating contact or engagement by surface 494 of valve element 492. Edge 496 is of a diameter larger than the diameter of inlet passage 372. There is provided second biasing means for urging check valve 468 into a position closing inlet 320. In particular, valve element surface 494 is biased into seating contact with edge 496 by the second biasing means acting on check valve 468 in the form of coil spring 498. One end of spring 498, i.e. the left-hand end as viewed in Figures 20—22, is seated in the shoulder provided by lands 480 on check valve body 470, and the opposite end of spring 498 is seated in the annular shoulder defined by surfaces 462 and 464 of armature body 134.

By way of example, in an illustrative pump, check valve body 470 is of titanium and has an overall axial length of about 1.78 mm (0.070 inch). Surface 472 of valve body 470 has a diameter of about 2.03 mm (0.080 inch) and bore 476 has a diameter of about 0.76 mm (0.030 inch) and an axial length of about 0.76 mm (0.030 inch). Each of the land portions 480a—480d has an axial length of about 1.02 mm (0.040 inch), each edge 482a—482d has a length of about 0.51 mm (0.020 inch) and edges 482a—482d lie about on a circle having a diameter of about 3.30 mm (0.130 inch). Recess 490 has a diameter of about 2.54 mm (0.100 inch) and an axial length of about 0.635 mm (0.025 inch). Valve element 492 is of Dow Corning Silastic material MDX4-4210 which is heat cured in place into recess 492. Surface 374 of inlet fitting 320 defines an angle of about 45 degrees with the housing longitudinal axis. Spring 498 is 0.005 diameter titanium wire with the spring inner diameter being about 4.62 mm (0.182 inch).

In operation, inlet 320 is connected to a source or supply of fluid to be pumped, and outlet 322 is connected to a point or location of use for the pumped fluid. The armature 420 is moved through a forward pumping stroke in response to electrically energization of electromagnet 380. One way of energizing magnet 380 is to charge a capacitor from a battery and then discharge that capacitor through coil 384. Other procedures can of course be employed for electrical energizing coil 384 in a known manner. Prior to electrical energization of magnet 380, armature 420 is in the rest position illustrated in Figure 20 where the check valve 468 is located with convex surface 494 seated against edge 496 surrounding the opening of passage 372 to block fluid communication from inlet 320 to the fluid receiving chamber 314. In the rest position of armature 420, pole face 426 is spaced from diaphragm 390 as shown in Figure 21 thereby defining the gap in the magnetic circuit. In the rest position this gap between pole face 426 and diaphragm 390 is of maximum length.

When coil 384 is electrically energized, the armature pole portion 424 is attracted toward magnet 380 thereby causing armature 420 to be drawn toward diaphragm 390. Electromagnetic flux travels through the magnetic circuit including the electromagnet core 382, magnet housing 386, rim 406 of body 400, the included portion of diaphragm 390 between housing 386 and rim 400, body 400, armature pole portion 424, and the gap between pole face 426 and diaphragm 390. As armature 420 is moved in the forward pumping stroke, i.e. in a direction to the left as viewed in Figures 20—22, fluid initially contained in the first pumping chamber 316, i.e. the region within housing inner surfaces 332 and between end face 354 of housing section 348 and face 404 of body 400, is forced by armature body portion 434 particularly end face 438 and flange 404 thereof, through the gap or clearance between armature pole portion 424 and passage 408 in body 400 into the second pumping chamber 318. Simultaneously, fluid contained in the second pumping chamber 318, i.e. the region between body 400, diaphragm 390 and pole portion 424, is thereby forced through passages 410 and 344 and out through the outlet 322. The clearance between outer surface 436 of body armature portion 134 and the inner surface 352 of housing section 348 is selected to be sufficiently small so that fluid leakage therebetween is relatively small during the forward pumping stroke of armature 420.

The check valve 468 slides freely with respect to the armature 420 and does not necessarily move when the armature 420 is drawn toward diaphragm 390. Such relative positions are illustrated, for example, in Figure 22. At rest, the surface 494 of check valve element 492 is held in contact with the valve seat defined by edge 496 by the spring 450 acting upon the armature 420 which is then in contact with the check valve body 470 as shown in Figure 20. When the armature 420 is drawn toward diaphragm 390, the force of spring 450 is no longer transferred to the check valve 468 and the force holding the valve surface 495 against the valve seat is decreased to that provided by spring 498, which generally provides a force less than that provided by spring 450. If armature 420 is drawn toward electromagnet 380, with sufficient velocity, pressure within the pump housing 312 between the end of armature body 434 and housing surface 360 and the check valve seat decreases to a level below the level at the pump inlet 320 and the net force due to fluid pressure from inlet 320 acting on the check valve 468 tends to move the check valve surface 494 away from the seat provided by edge 496. If the net force due to the fluid pressure exceeds that applied by the spring 498, then check valve 468 moves away from the valve seat and fluid flows into the pump body as illustrated in Figure 20. In fact, because the fluid is nearly incompressible the check valve 468 opens at approximately the same time that the armature 420 achieves enough velocity to force fluid out of the pump outlet 322. The forward pumping stroke of the armature 420 is completed when the pole face 426 approaches contact with the diaphragm 390. Actual contact may not be achieved since viscosity limits

14

**0 102 824**

outflow of the fluid between the pole face 426 and the diaphragm 390. When the armature velocity decreases to a level such that the displacement rate of the motion of the pole portion 424, no longer exceeds the leak rate between the outer surface 436 of armature body portion 434 and inner surface of housing section 348, the pressure within the pump housing 312 begins to increase. When the force due to the pressure difference across the check valve 468 no longer exceeds the force of spring 498, the check valve member moves toward the valve seat and prevents flow out of the inlet port 320 of the pump as illustrated, for example, in Figure 22.

When electrical excitation of coil 384 ceases, armature 420 is moved in the opposite direction, i.e. to the right as viewed in Figures 20—22, by the force of biasing spring 450 until the armature reaches the rest position as shown in Figure 21 with surface 494 seated on edge 496 surrounding the opening of passage 372. This motion is relatively slow since it is limited by the small leak rate of fluid between the outer surface 436 of armature body portion 434 and the inner surface 352 of housing section 348 at a pressure difference determined by the force applied by the spring 450. During the return stroke of armature 420 the check valve 468 is held against the valve seat primarily by the light spring 498 supplemented by the difference between the outlet and inlet pressures acting on the valve seat. When the return stroke has been completed the spring force is increased to that of spring 450. Thus, the average pumping rate which is determined by the rate of return of armature 420 to the rest position can be relatively slow, but such a pumping rate is called for in typical implantable drug delivery systems. Armature 420 then remains in the rest position of Figure 21 with inlet 320 closed and waiting for the next forward pumping stroke which occurs when magnet 380 is energized again.

Long term sealing is provided by the relatively stronger spring 450, and short term sealing while the armature 420 is forward is provided by the relatively weaker spring 498. As a result, there can be satisfactory sealing against the back flow when the pump is not in operation, while the pressure drop across the check valve 468 during the pump stroke is small.

The provisions of armature portion 434 and the housing sections 348, 350 at the inlet end of pump 310 results in a major decrease in the internal volume between the pump piston or armature 420 and the pump check valve 468. This reduction has the effect of making the pump less sensitive to the possible presence of a bubble in the fluid within the pump. As a result, the pump 319 is much easier to prime, and the use of carbon dioxide is normally not required for this purpose. When a bubble is introduced into the pump 310 along with the fluid stream, the flow rate decreases temporarily but recovers to its normal level as the bubble dissolves in the fluid. In addition, the volume per stroke of pump 310 is relatively less sensitive to back pressure.

The gap or clearance between armature pole portion 424 and passage 408 is relatively less critical to the extent that pump 310 relies on armature plunger portion 434 in addition to plunger portion 424 to provide the pressure increase. The Teflon coating 432 on the pole button or body 425 avoids high side forces on the pole button and the resulting drag as the pole button is pulled in a direction toward the armature spindle or shaft portion 422. The use of the Teflon coating 432, which is soft and somewhat uneven, makes it difficult to maintain a small clearance between the pole button 434 and the solenoid ring or body 400. In pump 310 greater clearance is allowable between these two parts. The requirement for close tolerance is shifted to two titanium parts which may not need to be coated, i.e. the armature portion 434 and the housing section 348.

The arrangement of armature plunger portions 434 and 424 in pumping chambers 316 and 318, respectively, provides two pumping elements in series. A higher pressure increase across pump 310 would appear to be possible and a bubble in one of the pumping chambers would affect one but not both pumping elements.

Thus, pump 310 of the present invention has the advantage of being relatively insensitive to the presence of bubbles in the fluid being handled, being relatively easy to prime, and providing an adequate fluid pressure increase across the pump sufficient for the uses intended. Pump 310 also has the advantage of operating at extremely low power levels, being compatible with drugs and similar liquids to be pumped, being electrically and magnetically efficient, and being small in size, light in weight and reliable in operation. In particular, the non-movable diaphragm 390 of titanium or like material provides a hermetic seal between the fluid in housing 312 and the electrical components associated with magnet 380. Having armature 420 immersed in the fluid makes operation of the pump nearly independent of ambient pressure. The initial condition of the pump when armature 420 is in the rest position of Figure 21 is that the fluid is at substantially the same pressure on opposite sides of the pump piston, i.e. in the receiving chamber 314 and in the pumping chambers 316 and 318.

The pump of the present invention is made electrically and magnetically efficient by minimizing the total gap within the magnetic circuit, by having the magnetic pole face 426 of relatively large surface area, and by having core 382 of relatively small diameter. In particular, there is a relatively large contact area at the interface between the axial end face of magnet housing 386 and diaphragm 390 and between diaphragm 390 and the axial end face of rim 406 of body 400 to minimize the effective air gap introduced by diaphragm 390 at this point in the magnetic circuit. Related to this is the need for welding diaphragm 390 to the band 392 and housing section 338 to achieve an hermetic seal between electromagnet 380 and the fluid containing region of housing 312 while at the same time not adversely affecting the magnetic circuit. In addition, there is a relatively large surface area along the gap between body 400 and pole portion 424 to

15

## 0 102 824

minimize the effective air gap introduced at this point in the magnetic circuit. The relatively small diameter of core 382 provides the necessary ampere turns with a minimum electrical resistance. The large area of pole face 426 provides a high magnetic force with a minimum number of ampere turns. Having the magnetic gap external to coil 384, i.e. between pole face 426 and diaphragm 390, allows the foregoing features to be achieved simultaneously.

**Claims**

1. An electromagnetic pump comprising:

a) an elongated housing (12 or 312) having an interior fluid containing region including a fluid receiving chamber (14 or 314) and a fluid pumping chamber (16 or 316) in fluid communication therewith, an inlet (18 or 320) in fluid communication with said receiving chamber and an outlet (20 or 322) in fluid communication with said pumping chamber;

b) check valve means (22) operatively associated with said fluid containing region for allowing fluid flow in a direction from said inlet (18 or 320) through said outlet (20 or 322) and blocking fluid flow in a direction from said outlet through said inlet;

c) electromagnet means (70 or 380) carried by said housing (12 or 312);

d) an armature (110 or 420) positioned in said fluid containing region of said housing (12 or 312) for movement along the longitudinal axis of said housing and having a pole portion (114 or 424) located for magnetic attraction by said electromagnet means and having a plunger portion in said pumping chamber (16 or 416) for forcing fluid out of said chamber through said outlet (20 or 322) said armature (110 or 420) being movably supported in said housing for movement from a rest position through a forward pumping stroke when attracted by said electromagnet means (70 or 380) to force fluid out of said pumping chamber (16 or 316) through said outlet (20 or 322) and for movement in an opposite direction through a return stroke back to said rest position; and

e) means for defining a magnetic circuit including said electromagnet means (70 or 380) and said armature (110 or 420) and a gap between said pole portion (114 or 424) of said armature and said electromagnet means for moving said armature toward said electromagnet means to close said gap in response to electrical energization of said electromagnet means; characterised in that:

f) said armature pole portion has a pole face (116 or 426) disposed in a plane substantially perpendicular to the direction of movement of said armature (110 or 420), said pole face being located to define said gap with said electromagnet means (70 or 380)s; and

g) said fluid containing region of said housing and said electromagnet means (70 or 380) are in axially spaced relation along said housing longitudinal axis and are separated by barrier means (80 or 390) of fluid impervious material disposed substantially perpendicular to said housing longitudinal axis, said fluid containing region, inlet (18 or 320), outlet (20 or 322) and armature (110 or 420) all being located axially on one side of said barrier means and said electromagnet means (70 or 380) being located axially on the opposite side of said barrier means.

2. A pump according to Claim 1, further including a body of magnetically permeable material (90 or 400) in said housing fluid containing region between said fluid receiving chamber (14 or 314) and said fluid pumping chamber, (16 or 316), said body defining a portion of said magnetic circuit, said body having a passage (98 or 408) therethrough for receiving said armature pole portion (114 or 424) in a movable, close-fitting relation whereby said armature (10 or 420) moves in said body during said forward and return strokes and said magnetic circuit is through said body and said armature pole portion.

3. A pump according to Claim 2, characterised in that the axial length of said passage (98 or 408) in said body (90 or 400) is relatively large as compared to the length of the space between the inner surface of said passage and the outer surface of said armature pole portion (114 or 424) to minimize the effective air gap defined therebetween and introduced in said magnetic circuit.

4. A pump according to Claim 2 or 3, characterised in that said barrier means (80 or 390) comprises a thin plate of metal compatible with the fluid, fixed between and providing an hermetic seal between said electromagnet means (70 or 380) and said fluid containing region of said housing; said body, of magnetically permeable material (90 or 400), said armature pole portion (114 or 424) and the portions of said barrier plate associated with said electromagnet.

5. A pump according to Claim 4, characterised in that the fluid pumping chamber (16 or 316) is defined between said barrier plate (80 or 390) and said body (90 or 400), said armature pole portion (114 or 424) moves in said pumping chamber and has a pole face (116 or 426) on the end thereof disposed toward said barrier plate, and said gap is defined between said armature pole face and said barrier plate, whereby as said gap is reduced during energization of said electromagnet means (70 or 380) causing the forward pumping stroke the movement of said armature pole face toward said barrier plate forces fluid out of said pumping chamber through said housing outlet (20 or 322).

6. A pump according to Claim 4, characterised in that said electromagnet means (70 or 380) includes a housing (76 or 386) of magnetically permeable material having an end face contacting one surface of said barrier plate (80 or 390), said body (90 or 400) has an end face contacting the other surface of said barrier plate, and the areas of contact of said end faces of said electromagnet housing and said body with said

16

# 0 102 824

surface of said plate are of sufficient size to minimize the effective air gap introduced by said plate in said magnetic circuit.

7. A pump according to any one of Claims 4 to 6, characterised in that the axial length of said passage (98 or 408) in said body (90 or 400) is relatively long as compared to the length of the space between the inner surface of said passage (98 or 408) and the outer surface of said armature pole portion (114 or 424) to minimize the effective air gap defined therebetween introduced in said magnetic circuit.

8. An electromagnetic pump including a housing (12 or 312) having an interior fluid containing region, an inlet (18 or 320) and an outlet (20 or 322) in fluid communication with said region, electromagnet means (70 or 380) carried by said housing and located external to said fluid containing region of said housing, and an armature (110 or 420) positioned in said fluid containing region of said housing having a pole portion (114 or 424) located for magnetic attraction by said electromagnet means causing movement of said armature to force fluid out of said region through said outlet, characterised in that the pump includes:

a) barrier means (80 or 390) of fluid-impervious material separating said electromagnet means (70 or 380) and said fluid containing region of said housing (12 or 312), said fluid containing region, inlet (18 or 320), outlet (20 or 322) and armature (110 or 420) all being located axially on one side of said barrier means (80 or 390) and said electromagnet means (70 or 380) being located axially on the opposite side of said barrier means.

b) a body of magnetically permeable material (90 or 400) in said housing fluid containing region and having a passage (98 or 408) therethrough for receiving said armature pole portion (114 or 424) in a movable, close-fitting relation; and

c) means for defining a magnetic circuit including said electromagnet means (70 or 380), said body (90 or 400), the included portions of said barrier means (80 or 390) between said electromagnet means and said body, said armature pole portion (114 or 424) and a gap in said fluid containing region of said housing between said armature pole portion and said electromagnet means for moving said armature toward said electromagnet means to close said gap in response to electrical energization of said electromagnet means.

9. An electromagnetic pump according to Claim 8 characterised in that said barrier means comprises a thin metal plate (80 or 390) fixed between and providing an hermetic seal between said electromagnet means (70 or 380) and said fluid containing region of said housing (12 or 312) and wherein said electromagnet means (70 or 380) includes a housing (76 or 386) of magnetically permeable material having an end face contacting one surface of said barrier plate, said body (90 or 400) has an end face contacting the outer surface of said barrier plate, and the areas of contact of said end faces of said electromagnet housing and said body with said surface of said plate are are of sufficient size to minimize the effective air gap introduced by said plate in said magnetic circuit.

10. A pump according to Claim 8 or Claim 9, characterised in that the axial length of said passage (98 or 408) in said body (90 or 400) is relatively long as compared to the length of the space between the inner surface of said passage and the outer surface of said armature pole portion (114 or 424) minimize the effective air gap defined therebetween introduced in said magnetic circuit.

11. An electromagnetic pump including a housing (12 or 312) having an interior fluid containing region, an inlet (18 or 320) and an outlet (20 or 322) in fluid communication with said region, electromagnet means (70 or 380) carried by said housing and located external to said fluid-containing region, and an armature (110 or 420) positioned in said housing for movement from a rest position through a forward pumping stroke when attracted by said electromagnet means to force fluid out said fluid containing region through said outlet and for movement in an opposite direction through a return stroke back to said rest position, characterised in that:

a) check valve means (170 or 468) are operatively coupled to said armature (110 or 420) and located in said fluid containing region of said housing (12 or 312) for closing said inlet (18 or 320) when said armature is in said rest position and for opening said inlet after said armature begins movement associated with said forward pumping stroke, said check valve means allowing fluid flow in a direction from said inlet through said outlet (20 or 322) and blocking fluid flow in a direction from said outlet through said inlet; and

b) said fluid containing region of said housing and said electromagnet means (70 or 380) are in axially spaced relation along said housing longitudinal axis and are separated by barrier means (80 or 390) of fluid impervious material disposed substantially perpendicular to said housing longitudinal axis, said fluid containing region, inlet (18 or 320) outlet (20 or 322) and armature (40 or 420) all being located axially on one side of said barrier means and said electromagnet means being located axially on the opposite side of said barrier means.

12. An electromagnetic pump according to Claim 11, characterised in that said check valve means (170 or 468) comprises a valve member (172 or 470) movably carried by said armature (110 or 420), and further including:

a) first biasing means (200 or 450) for urging said armature (110 or 420) toward said rest position;

b) second biasing means (204 or 498) for urging said valve member (172 or 470) toward a position closing said inlet (18 or 320); and

c) the force provided by said first biasing means (200 or 450) being greater than the force provided by said second biasing means (204 or 498).

13. An electromagnetic pump according to Claim 11 or Claim 12, characterised in that:

a) said fluid containing region includes a fluid receiving chamber (314), a first fluid pumping chamber

17

(316) in fluid communication therewith, a second fluid pumping chamber (318) in fluid communication with said first pumping chamber, said inlet (320) being in fluid communication with said receiving chamber and said outlet (322) being in fluid communication with said second pumping chamber;

b) said armature (420) is positioned in said fluid containing region of said housing (312) and has a pole portion (424) located for magnetic attraction by said electromagnet means (380) and a first plunger portion (434) in said first pumping chamber (316) for forcing fluid out of said first chamber into said second pumping chamber (318) and a second plunger portion (424) in said second pumping chamber (318) for forcing fluid out of said second chamber through said outlet (322); said armature being movably supported in said housing for movement from a rest position through a forward pumping stroke when attracted by said electromagnet means to force fluid from said pumping chambers (316 and 318) through said outlet (322) and for movement in an opposite direction through a return stroke back to said rest position; and

c) means are provided for defining a magnetic circuit including said electromagnetic means (380) and said armature (420) and a gap between said pole portion (424) of said armature and said electromagnet means for moving said armature toward said electromagnetic means to close said gap in response to electrical energization of said electromagnetic means.

14. A pump according to Claim 13, characterised in that said check valve means (468) is in relatively close-fitting relation to the portion of said housing (312) defining said fluid receiving chamber (314), said check valve means is closely coupled to and located closely adjacent said armature (420), and the portion of said armature adjacent said check valve means is in relatively close-fitting relation to said housing thereby decreasing the volume of said interior fluid containing region between said armature and said check valve means.

15. A pump according to Claim 14, characterised in that the cross-sectional dimension of said armature portion adjacent said check valve means (468) and the cross-sectional size of the associated portion of said housing (312) are so related to define a close tolerance space therebetween serving as a leakage path for fluid from said first pumping chamber (316) to said receiving chamber (314) during movement of said armature through said return stroke.

16. A pump according to Claim 13, characterised in that said housing (312) includes a cylindrical portion (396) adjacent said inlet (320) and having a first inner diameter section (358) containing said check valve means (468) in relatively close-fitting relation and a second, smaller inner diameter section (352) containing said first plunger portion (434) of said armature (420) in relatively close-fitting relation said check valve means being closely coupled to and located closely adjacent said armature, thereby decreasing the volume of said interior fluid containing region between said armature and said check valve means.

17. A pump according to Claim 16, characterised in that the inner diameter of said second housing section (352) and the outer diameter of said armature first plunger portion (434) are so related to define a close tolerance space therebetween serving as a leakage path for fluid from said first pumping chamber (316) to said receiving chamber (314) during movement of said return stroke.

18. A pump according to any one of Claims 13 to 17, further including a body (400) of magnetically permeable material in said housing fluid containing region between said first and second fluid pumping chambers, (316 and 318), said body defining a portion of said magnetic circuit, said body having a passage (408) therethrough for receiving said second armature plunger portion (424) in a movable, relatively close-fitting relation whereby said armature (428) moves in said body (400) during said forward and return strokes and said magnetic circuit is through said body and said second armature plunger portion (424).

19. A pump according to any one of Claims 13 or 18, further including biasing means (450) in said housing for urging said armature (420) toward said rest portion.

20. A pump according to Claim 19, further including:

a) first biasing means (450) for urging said armature (420) toward said rest position;

b) second biasing means (498) for urging said check valve means (468) toward a position closing said inlet (320); and

c) the force provided by said first biasing means (450) being greater than the force provided by said second biasing means (498).

21. A pump according to Claim 19, further including:

a) first biasing means (450) operatively associated with said armature (420) for providing long term sealing of said pump inlet (320);

b) second biasing means (498) operatively associated with said check valve means (468) for providing short term sealing of said pump inlet (320); and

c) the force provided by said second biasing means (498) being weaker than the force provided by said first biasing means (450);

d) whereby sealing against back flow is provided when said pump is not in operation while pressure drop across said check valve means is relatively small during the pumping stroke.

**Patentansprüche**

1. Elektromagnetische Pumpe mit

a) einem länglichen Gehäuse (12 oder 312) mit einem inneren, Fluid enthaltenden Bereich, welcher eine Fluid-Aufnahmekammer (14 oder 314) und eine damit in Verbindung stehende Fluid-Pumpkammer (16 oder 316), einen mit der Aufnahmekammer in Fluidverbindung stehenden Einlaß (18 oder 320) und einen mit der Pumpkammer in Fluidverbindung stehenden Auslaß (20 oder 322) aufweist;

b) einem Rückschlagventil (22), welches wirksam dem Fluid enthaltenden Bereich zugeordnet ist, um eine Fluidströmung in einer Richtung von dem Einlaß (18 oder 320) durch den Auslaß (20 oder 322) zuzulassen und um eine Fluidströmung in einer Richtung von dem Auslaß durch den Einlaß zu sperren;

c) einem Elektromagneten (70 oder 380), der von dem Gehäuse (12 oder 312) getragen ist;

d) einem Anker (110 oder 420), welcher in dem Fluid enthaltenden Bereich des Gehäuses (12 oder 312) für eine Bewegung entlang der Längsachse des Gehäuses angeordnet ist, und der einen Polteil (114 oder 424), welcher für eine magnetische Anziehung durch den Elektromagneten örtlich festgelegt ist, und einen Plungerteil in der Pumpkammer (16 oder 416) hat, um Fluid aus der Kammer durch den Auslaß (20 oder 322) zu drücken, wobei der Anker (110 oder 420) in dem Gehäuse für eine Bewegung aus einer Ruhestellung über einen Vorwärtspumphub, wenn er durch den Elektromagneten (70 oder 380) angezogen worden ist, um Fluid aus der Pumpkammer (16 oder 316) durch den Auslaß (20 oder 322) zu drücken, und für eine Bewegung in einer entgegengesetzten Richtung über einen Rückkehrhub zurück in die Ruhestellung beweglich gehalten ist, und

e) einer Einrichtung zum Festlegen eines magnetischen Kreises, welcher den Elektromagneten (70 oder 380) und den Anker (110 oder 420) sowie einen Spalt zwischen dem Polteil (114 oder 424) des Ankers und des Elektromagneten aufweist, um den Anker in Richtung des Elektromagneten zu bewegen, um dadurch den Spalt entsprechend einer elektrischen Erregung des Elektromagneten zu schließen, dadurch gekennzeichnet, daß

f) der Anker-Polteil eine Polfläche (116 oder 426) hat, welche in einer Ebene angeordnet ist, welche genau senkrecht zu der Bewegungsrichtung des Ankers (110 oder 420) ist, wobei die Polfläche entsprechend fixiert ist, um den Spalt bezüglich des Elektromagneten (70 oder 380) festzulegen, und

g) der Fluid enthaltende Bereich des Gehäuses und der Elektromagnet (70 oder 380) in axialer Beziehung entlang der Gehäuse-Längsachse im Abstand voneinander angeordnet sind und durch eine Sperreinrichtung (80 oder 390) aus fluidundurchlässigem Material getrennt sind, welche genau senkrecht zu der Gehäuselängsachse angeordnet ist, wobei der Fluid enthaltende Bereich, der Einlaß (18 oder 320), der Auslaß (20 oder 322) und der Anker (110 oder 420) alle axial auf einer Seite der Sperreinrichtung festgelegt sind, und der Elektromagnet (70 oder 380) in axialer Richtung auf der anderen Seite der Sperreinrichtung festgelegt ist.

2. Pumpe nach Anspruch 1, gekennzeichnet durch einen Körper aus magnetisch durchlässigem Material (90 oder 400) in dem Fluid enthaltenden Bereich in dem Gehäuse zwischen der Fluid-Aufnahmekammer (14 oder 314) und der Fluidpumpkammer (16 oder 316), wobei der Körper einen Teil des magnetischen Kreises festlegt und einen Durchlaß (98 oder 408) aufweist, um den Anker-Polteil (114 oder 424) bewegbar und satt anliegend aufzunehmen, wobei sich der Anker (10 oder 420) in dem Körper während der Vorwärts- und Rückkehrhübe bewegt und der magnetische Kreis durch den Körper und den Anker-Polteil hindurchgeht.

3. Pumpe nach Anspruch 2, dadurch gekennzeichnet, daß die axiale Länge des Durchlasses (98 oder 408) in dem Körper (90 oder 400) im Vergleich zu der Länge des Zwischenraums zwischen der Innenfläche des Durchlasses und der Außenfläche des Anker-Polteils (114 oder 424) verhältnismäßig groß ist, um den wirksamen Luftspalt zu minimieren, der dazwischen fesgelegt ist und in dem magnetischen Kreis eingebracht ist.

4. Pumpe nach Anspruch 2 oder 3, dadurch gekennzeichnet, daß die Sperrvorrichtung (80 oder 390) eine dünne Platte aus einem mit dem Fluid verträglichen Metall aufweist, welche dazwischen befestigt ist und eine hermetische Abdichtung zwischen dem Elektromagneten (70 oder 380) und dem Fluid enthaltenden Bereich des Gehäuses, dem Körper aus magnetisch durchlässigem Material (90 oder 400), dem Anker-Polteil (114 oder 424) und den Teilen der Sperrplatte, welche dem Elektromagneten zugeordnet ist, aufweist.

5. Pumpe nach Anspruch 4, dadurch gekennzeichnet, daß die Fluid-Pumpkammer (16 oder 316) zwischen der Sperrplatte (80 oder 390) und dem Körper (90 oder 400) festgelegt ist, der Anker-Polteil (114 oder 424) sich in der Pumpkammer bewegt und eine Polfläche (116 oder 426) an dem Ende aufweist, das in Richtung der Sperrplatte angeordnet ist, und der Spalt zwischen der Anker-Polfläche und der Sperrplatte festgelegt ist, wobei, wenn der Spalt durch eine Erregung des Elektromagneten (70 oder 380) verringert wird, aufgrund des Vorwärts-Pumphubs durch die Bewegung der Anker-Polfläche in Richtung der Sperrplatte Fluid aus der Pumpkammer durch den Gehäuseauslaß (20 oder 322) gedrückt wird.

6. Pumpe nach Anspruch 4, dadurch gekennzeichnet, daß der Elektromagnet (70 oder 380) ein Gehäuse (76 oder 386) aus magnetisch permeablen Material aufweist, wobei eine Endfläche eine Oberfläche der Sperrplatte (80 oder 390) berührt, der Körper (90 oder 400) eine Endfläche hat, welche die andere Oberfläche der Sperrplatte berührt, und die Berührungsflächen der Endflächen des Elektromagnetengehäuses und des Körpers mit der Oberfläche der Platte eine ausreichende Größe bahen, um den wirksamen Luftspalt zu minimieren, welcher durch die Platte in den magnetischen Kreis eingebracht ist.

7. Pumpe nach einem der Ansprüche 4 bis 6, dadurch gekennzeichnet, daß die axiale Länge des Durchlasses (98 oder 408) in dem Körper (90 oder 400) im Vergleich zu der Länge des Zwischenraums zwischen der Innenfläche des Durchlasses (98 oder 408) und der Außenfläche des Anker-Polteils (114 oder

19

# 0 102 824

424) verhältnismäßig lang ist, um den wirksamen, dazwischen festgelegten Luftspalt in dem magnetischen Kreis zu minimieren.

8. Elektromagnetische Pumpe mit einem Gehäuse (12 oder 312) mit einem inneren Fluid enthaltenden Bereich, einem Einlaß (18 oder 320) und einem Auslaß (20 oder 322) welche in Fluidverbindung mit dem Bereich stehen, mit einem Elektromagneten (70 oder 380), der durch das Gehäuse getragen ist und außerhalb des Fluid enthaltenden Bereichs des Gehäuses festegelegt ist, und mit einem Anker (110 oder 420), der in dem Fluid enthaltenden Bereich des Gehäuses angeordnet ist und einen Polteil (114 oder 424) hat, welcher für eine magnetische Anziehung durch den Elektromagneten örtlich festgelegt ist, um durch eine Bewegung des Ankers Fluid aus dem Bereich durch den Auslaß zu drücken, dadurch gekennzeichnet, daß die Pumpe aufweist:

a) eine Sperreinrichtung (80 oder 390) aus fluid-undurchlässigem Material, wodurch der Elektromagnet (70 oder 380) und der Fluid enthaltende Bereich des Gehäuses (12 oder 312) getrennt werden, wobei der Fluide enthaltende Bereich, der Einlaß (18 oder 320), der Auslaß (20 oder 322) und der Anker (110 oder 420) alle in axialer Richtung auf einer Seite der Sperreinrichtung (80 oder 390) festgelegt sind, und der Elektromagnet (70 oder 380) in axialer Richtung auf der anderen Seite der Sperreinrichtung festgelegt ist;

b) einen Körper aus magnetisch permeablem Material (90 oder 400) in dem Fluid enthaltenden Gehäusebereich mit einem durchgehenden Durchlaß (98 oder 408), um den Anker-Polteil (114 oder 424) bewegbar, jedoch satt anliegend aufzunehmen, und

c) eine Einrichtung zum Festlegen eines magnetischen Kreises, welcher den Elektromagneten (70 oder 380), den Körper (90 oder 400), die entsprechenden Teile der Sperreinrichtung (80 oder 390) zwischen dem Elektromagneten und dem Körper, den Anker-Polteil (114 oder 424) und einen Spalt in dem Fluid enthaltenden Bereich des Gehäuses zwischen dem Anker-Polteil und dem Elektromagneten aufweisen, um den Anker in Richtung des Elektromagneten zu bewegen, um dadurch den Spalt entsprechend einer elektrischen Erregung des Elektromagneten zu schließen.

9. Elektromagnetische Pumpe nach Anspruch 8, dadurch gekennzeichnet, daß die Sperreinrichtung eine dazwischen befestigte, dünne Metallplatte (80 oder 390) aufweist, welche eine hermetische Abdichtung zwischen dem Elektromagneten (70 oder 380) und dem Fluid enthaltenden Bereich des Gehäuses (12 oder 312) aufweist, und daß der Elektromagnet (70 oder 380) ein Gehäuse (76 oder 386) aus magnetisch permeablem Material aufweist, von welchem eine Endfläche eine Oberfläche der Sperrplatte berührt, daß der Körper (90 oder 400) eine Endfläche hat, welche die äußere Oberfläche der Sperrplatte berührt, und die Berührungsflächen der Endflächen des Elektromagnetgehäuses und des Körpers mit der Oberfläche der Platte eine ausreichende Größe haben, um den wirksamen Luftspalt zu minimieren, welcher durch die Platte in den magnetischen Kreis eingebracht ist.

10. Pumpe nach Anspruch 8 oder 9, dadurch gekennzeichnet, daß die axiale Länge des Durchlasses (98 oder 408) in dem Körper (90 oder 400) im Vergleich zu der Länge des Zwischenraums zwischen der Innenfläche des Durchlasses und der Außenfläche des Anker-Polteils (114 oder 424) verhältnismäßig lang ist, um den dazwischen festgelegten, wirksamen Luftspalt in dem magnetischen Kreis zu minimieren.

11. Elektromagnetische Pumpe mit einem Gehäuse (12 oder 312) mit einem inneren, Fluid enthaltenden Bereich, einem Einlaß (18 oder 32) und einem Auslaß (20 oder 322), die mit dem Bereich in Fluidverbindung stehen, mit einem Elektromagneten (70 oder 380), der von dem Gehäuse getragen ist und außerhalb des Fluid enthaltenden Bereichs festgelegt ist, und mit einem Anker (310 oder 420), der in dem Gehäuse für eine Bewegung aus einer Ruhestellung über einen Vorwärts-Pumphub, wenn er mittels des Elektromagneten angezogen worden ist, um Fluid aus dem Fluid enthaltenden Bereich durch den Auslaß zu drücken, und für eine Bewegung in einer entgegengesetzten Richtung über einen Rückkehrhub zurück in die Ruhestellung positioniert ist, dadurch gekennzeichnet, daß

a) eine Rückschlagventileinrichtung (170 oder 468) wirksam mit dem Anker (110 oder 420) verbunden ist und in dem Fluid enthaltenden Bereich des Gehäuses (12 oder 312) festgelegt ist, um den Einlaß (18 oder 320) zu schließen, wenn sich der Anker in der Ruhestellung befindet, und um den Einlaß zu öffnen, nachdem der Anker eine dem Vorwärtspumphub zugeordnete Bewegung beginnt, wobei die Rückschlagventileinrichtung eine Fluidströmung in einer Richtung von dem Einlaß durch den Auslaß (20 oder 322) zuläßt und eine Fluidströmung in einer Richtung von dem Auslaß durch den Einlaß hindurch sperrt, und

b) der Fluid enthaltende Bereich des Gehäuses und der Elektromagnet (70 oder 380) in axialer Richtung im Abstand entlang der Gehäuselängachse angeordnet sind und durch eine Sperreinrichtung (80 oder 390) aus einem fluid-undurchlässigen Material getrennt sind, welche genau senkrecht zu der Gehäuselängsachse angeordnet ist, wobei der Fluid enthaltende Bereich, der Einlaß (18 oder 320), der Außlaß (20 oder 322) und der Anker (40 oder 420) alle in axialer Richtung auf einer Seite der Sperreinrichtung und der Elektromagnet in axialer Richtung auf der anderen Seite der Sperreinrichtung festgelegt sind.

12. Elektromagnetische Pumpe nach Anspruch 11, dadurch gekennzeichnet, daß die Sperrventileinrichtung (170 oder 468) ein Ventilteil (172 oder 470) aufweist, das durch den Anker (110 oder 420) beweglich getragen ist, und ferner aufweist

a) eine erste Vorspanneinrichtung (200 oder 450), um den Anker (110 oder 420) in Richtung der Ruhestellung zu drücken;

20

b) eine zweite Vorspanneinrichtung (204 oder 498), um das Ventilteil (172 oder 460) in Richtung einer Stellung zu drücken, in welcher der Einlaß (18 oder 320) geschlossen ist, und

c) die durch die erste Vorspanneinrichtung (200 oder 450) erzeugte Kraft größer als die durch die zweite Vorspanneinrichtung (204 oder 498) erzeugte Kraft ist.

13. Elektromagnetische Pumpe nach Anspruch 11 oder 12, dadurch gekennzeichnet, daß

a) der Fluid enthaltende Bereich eine Fluid-Aufnahmekammer (314), eine erste, damit in Verbindung stehende Fluid-Pumpkammer (316) und eine zweite mit der ersten Pumpkammer in Fluid-Verbindung stehende Fluid-Pumpkammer (318) aufweist, wobei der Einlaß (320) mit der Aufnahmekammer und der Auslaß (322) mit der zweiten Pumpkammer in Fluidverbindung steht;

b) der Anker (420) in dem Fluid enthaltenden Bereich des Gehäuses positioniert ist und einen Polteil (424), welcher für eine magnetische Anziehung durch den Elektromagneten (380) festgelegt ist, und einen ersten Plungerteil (434) in der ersten Pumpkammer (316), um Fluid aus der ersten Kammer in die zweite Pumpkammer (318) zu drücken, und einen zweiten Plungerteil (424) in der zweiten Pumpkammer (318) hat, um Fluid aus der zweiten Kammer durch den Auslaß (322) zu drücken, wobei der Anker in dem Gehäuse für eine Bewegung aus einer Ruhestellung über einen Vorwärts-Pumphub, wenn er durch den Elektromagneten angezogen worden ist, um Fluid aus den Pumpkammern (316 und 318) durch den Auslaß (322) zu drücken, und für eine Bewegung in einer entgegengesetzten Richtung über einen Rückkkerhrhub zurück in die Ruhestellung beweglich gehaltert ist, und

c) Einrichtungen vorgesehen sind, um einen magnetischen Kreis, welcher den Elektromagneten (380) und den Anker (420) sowie einen Spalt zwischen dem Polteil (424) des Ankers und dem Elektromagneten einschließt, um den Anker in Richtung des Elektromagneten zu bewegen, um den Spalt entsprechend einer elektrischen Erregung des Elektromagneten zu schließen.

14. Pumpe nach Anspruch 13, dadurch gekennzeichnet, daß die Rückschlagventileinrichtung (468) verhältnismäßig satt an dem Teil des Gehäuses anliegt, welcher die Fluid-Aufnahmekammer (314) festlegt, die Rückschlagventileinrichtung eng mit dem Anker (420) verbunden und angrenzend an den Anker (420) festgelegt ist, und der Teil des Ankers, welcher der Rückschlagventileinrichtung benachbart ist, verhältnismäßig satt an dem Gehäuse anliegt, um dadurch das Volumen des inneren, Fluid enthaltenden Bereichs zwischen dem Anker und der Rückschlagventileinrichtung zu verringern.

15. Pumpe nach Anspruch 14, dadurch gekennzeichnet, daß die Querschnittsabmessung des Ankerteils, welcher der Rückschlagventileinrichtung (468) benachbart ist und die Querschnittsgröße des zugeordneten Teils des Gehäuses (312) eine entsprechende Beziehung zueinander haben, um einen Zwischenraum. enger Toleranz festzulegen, welcher als eine Leckbahn für das Fluid von der ersten Pumpkammer (316) zu der Aufnahmekammer (314) während einer Bewegung des Ankers über den Rückkehrhub dient.

16. Pumpe nach Anspruch 13, dadurch gekennzeichnet, daß das Gehäuse (312) angrenzend an den Einlaß (320) einen zylindrischen Teil (396) aufweist und einen ersten Abschnitt (358), welcher im Inneren verhältnismäßig satt anliegend die Rückschlagventileinrichtung (468) enthält, und einen zweiten Abschnitt (352) mit einem kleineren Innendurchmesser aufweist, welcher den ersten Plungerteil (434) des Ankers (420) verhältnismäßig satt anliegend enthält, wobei die Rückschlagventileinrichtung eng mit dem Anker verbunden und eng angrenzend an den Anker festgelegt ist, um dadurch das Volumen des Inneren, Fluid enthaltenden Bereichs zwischen dem Anker und der Rückschlagventileinrichtung zu verringern.

17. Pumpe nach Anspruch 16, dadurch gekennzeichnet, daß der Innendurchmesser des zweiten Gehäuseabschnitts (352) und der Außendurchmesser des ersten Anker-Plungerteils (434) eine entsprechende Beziehung haben, um einen Zwischenraum mit geringer Toleranz festzulegen, welcher als eine Leckbahn für das Fluid während einer Bewegung des Rückkehrhubs von der ersten Pumpkammer (316) zu der Aufnahmekammer (314) dient.

18. Pumpe nach einem der Ansprüche 13 bis 17, gekennzeichnet, durch einen Körper (400) aus magnetisch permeablem Material in dem Fluid enthaltenden Gehäusebereich zwischen der ersten und zweiten Fluid-Pumpkammer (316 und 318), wobei der Körper einen Teil des magnetischen Kreises festlegt und einen durchgehenden Durchlaß (408) hat, um den zweiten Anker-Plungerteil (424) beweglich und verhältnismäßig satt an liegende aufzunehmen, wobei sich der Anker in dem Körper (400) während der Vorwärs- und Rückkehrhube bewegt und der magnetische Kreis durch den Körper und den zweiten Anker-Plungerteil (424) geht.

19. Pumpe nach einem der Ansprüche 13 oder 18, gekennzeichnet, durch eine Vorspanneinrichtung (54) in dem Gehäuse, um den Anker (420) in Richtung der Ruhestellung zu drücken.

20. Pumpe nach Anspruch 19, gekennzeichnet, durch

a) eine erste Vorspanneinrichtung (450), um den Anker (420) in Richtung der Ruhestellung zu drücken;

b) eine zweite Vorspanneinrichtung (498), um die Rückschlagventileinrichtung (468) in Richtung einer Stellung nahe dem Einlaß (320) zu drücken, und

c) die durch die erste Vorspanneinrichtung (520) erzeugte Kraft größer als die von der zweiten Vorspanneinrichtung (498) erzeugte Kraft ist.

21. Pumpe nach Anspruch 19, gekennzeichnet durch

a) eine erste Vorspanneinrichtung (450), welche wirksam dem Anker (420) zugeordnet ist, um eine langfristige Abdichtung des Pumpeneinlasses (320) zu schaffen;

# 0 102 824

b) eine zweite Vorspanneinrichtung (498), die wirksam der Rückschlagventileinrichtung (468) zugeordnet ist, um eine kurzzeitige Abdichtung des Pumpeneinlasses (320) zu schaffen, und

c) die durch die zweite Vorspanneinrichtung (498) erzeugte Kraft größer als die durch die ersten Vorspanneinrichtung (450) erzeugte Kraft ist,

d) wobei eine Abdichtung gegen eine Rückströmung vorgesehen ist, wenn die Pumpe nicht im Betrieb ist, während ein Druckabfall an der Rückschlagventileinrichtung während des Pumphubs verhältnismäßig klein ist.

**Revendications**

1. Pompe électromagnétique comprenant:

(a) un carter allongé (12 ou 312) ayant une région intérieure de contenance de fluide qui comporte une chambre de réception de fluide (14 ou 314) et une chambre de pompage de fluide (16 ou 316) en communication de fluide avec elle, une entrée (18 ou 320) en communication de fluide avec ladite chambre de réception et une sortie (20 ou 322) en communication de fluide avec ladite chambre de pompage;

(b) des moyens à clapet anti-retour (22) fonctionnellement associés à ladite région de contenance de fluide pour permettre l'écoulement de fluide dans une direction à partir de ladite entrée (18 ou 320) à travers ladite sortie (20 ou 322) et pour empêcher l'écoulement du fluide dans une direction à partir de ladite sortie à travers ladite entrée;

(c) des moyens électro-magnétiques (70 ou 380) portés par le dit carter (12 ou 312);

(d) une armature (110 ou 420) située dans ladite région de contenance de fluide dudit carter (12 ou 312) pour un mouvement suivant l'axe longitudinal dudit carter et comportant une partie polaire (114 ou 424) située pour une attraction magnétique par lesdits moyens électromagnétiques et comportant une partie formant plongeur dans ladite chambre de pompage (16 ou 416) pour refouler le fluide de ladite chambre à travers ladite sortie (20 ou 322), ladite armature (110 ou 420) étant supportée de façon mobile dans ledit carter pour un mouvement à partir d'une position de repos et suivant une course de pompage vers l'avant, lorsqu'elle est attirée par les dits moyens électro-magnétiques (70 ou 380) afin de refouler le fluide de ladite chambre de pompage (16 ou 316) à travers ladite sortie (20 ou 322), et pour un mouvement dans une direction opposée suivant une course de retour à ladite position de repos; et

(e) des moyens pour définir un circuit magnétique comprenant lesdits moyens électro-magnétiques (70 ou 380) et ladite armature (110 ou 420) et un intervalle ou entrefer entre ladite partie polaire (114 ou 424) de ladite armature et lesdits moyens électro-magnétiques pour déplacer ladite armature vers lesdits moyens électromagnétiques afin de fermer ledit intervalle en réponse à l'excitation électrique desdits moyens électro-magnétiques; caractérisé en ce que:

(f) ladite partie polaire de l'armature présente une face polaire (116 ou 426) disposée dans un plan sensiblement perpendiculaire à la direction de mouvement de ladite armature (110 ou 420), ladite face polaire étant située de manière à définir ledit intervalle avec lesdits moyens électro-magnétiques (70 ou 380); et

(g) ladite région de contenance de fluide dudit carter et lesdits moyens électro-magnétiques (70 ou 380) sont axialement espacés le long dudit axe longitudinal du carter et sont séparés par des moyens de barrière (80 ou 390) en matière imperméable au fluide, disposés sensiblement perpendiculairement audit axe longitudinal du carter, ladite région de contenance de fluide, l'entrée (18 ou 320), la sortie (20 ou 322) et l'armature (110 ou 420) étant toutes situées axialement d'un côté desdits moyens de barrière et lesdits moyens électromagnétiques (70 ou 380) étant situés axialement sur le côté opposé desdits moyens de barrière.

2. Pompe suivant la revendication 1, comprenant en outre un corps de matière magnétiquement perméable (90 ou 400) dans ladite région de contenance de fluide du carter entre ladite chambre de réception de fluide (14 ou 314) et ladite chambre de pompage de fluide (16 ou 316), ce corps définissant une partie dudit circuit magnétique, ledit corps étant traversé par un passage (98 ou 408) pour recevoir la dite partie polaire d'armature (114 ou 424) en ajustement étroit et mobile de sorte que ladite armature (10 ou 420) se déplace dans ledit corps pendant lesdites courses avant et de retour et ledit circuit magnétique s'effectue à travers ledit corps et ladite partie polaire d'armature.

3. Pompe suivant la revendication 2, caractérisée en ce que la longueur axiale dudit passage (98 ou 408) dans ledit corps (90 ou 400) est relativement grande par rapport à la longueur de l'espace entre la surface intérieure dudit passage et la surface extérieure de ladite partie polaire d'armature (114 ou 424), afin de minimiser l'intervalle d'air effectif défini entre elles et introduit dans ledit circuit magnétique.

4. Pompe suivant la revendication 2 ou 3, caractérisée en ce que lesdits moyens de barrière (80 ou 390) comprennent une plaque mince en métal compatible avec le fluide, fixée entre et constituant une fermeture hermétique entre lesdits moyens électro-magnétiques (70 ou 380) et la dite région de contenance de fluide dudit carter, ledit corps en matière magnétiquement perméable (90 ou 400), la dite partie polaire d'armature (114 ou 424) et les parties de ladite plaque de barrière associées dudit électro-aimant.

5. Pompe suivant la revendication 4, caractérisée en ce que la chambre de pompage de fluide (16 ou 316) est définie entre ladite plaque de barrière (80 ou 390) et le dit corps (90 ou 400), ladite partie polaire d'armature (114 ou 424) se déplace dans ladite chambre de pompage et présente une face polaire (116 ou 426) sur son extrémité disposée vers ladite plaque de barrière, et le dit intervalle est défini entre ladite face

**0 102 824**

polaire d'armature et ladite plaque de barrière, de sorte que, lorsque ledit intervalle est réduit pendant l'excitation desdits moyens électromagnétiques (70 ou 380) engendrant la course de pompage vers l'ayant, le mouvement de ladite face polaire d'armature vers ladite plaque de barrière refoule le fluide à l'extérieur de ladite chambre de pompage à travers ladite sortie de carter (20 ou 322).

6. Pompe suivant la revendication 4, caractérisée en ce que lesdits moyens électro-magnétiques (70 ou 380) comprennent une enveloppe (76 ou 386) en matière magnétiquement perméable présentant une face d'extrémité en contact avec une surface de ladite plaque de barrière (80 ou 390), ledit corps (90 ou 400) présente une face d'extrémité en contact avec l'autre surface de ladite plaque de barrière, et les zones de contact des dites faces d'extrémité de la dite enveloppe d'électroaimant et dudit corps avec ladite surface de ladite plaque ont une dimension suffisante pour minimiser l'intervalle d'air effectif introduit par ladite plaque dans ledit circuit magnétique.

7. Pompe suivant l'une quelconque des revendications 4 à 6, caractérisée en ce que la longueur axiale dudit passage (98 ou 408) dans ledit corps (90 ou 400) est relativement longue par rapport à la longeur de l'espace défini entre la surface intérieure dudit passage (98 ou 408) et la surface extérieure de ladite partie polaire d'armature (114 ou 424) afin de minimiser l'intervalle d'air effectif défini entre ces surfaces et introduit dans ledit circuit magnétique.

8. Pompe électromagnétique comprenant un carter (12 ou 312) qui comporte une région intérieure de contenance de fluide, une entrée (18 ou 320) et une sortie (20 ou 322) en communication de fluide avec ladite région, des moyens électromagnétiques (70 ou 380) portés par ledit carter et situés à l'extérieur de ladite région de contenance de fluide dudit carter, et une armature (110 ou 420) située dans ladite région de contenance de fluide dudit carter et comportant une partie polaire (114 ou 424) situé pour une attraction magnétique par lesdits moyens électromagnétiques engendrant un mouvement de ladite armature de manière à refouler le fluide à l'extérieur de ladite région à travers ladite sortie, caractérisée, en ce que la pompe comprend:

(a) des moyens de barrière (80 ou 390) en matière imperméable au fluide qui séparent lesdits moyens électromagnétiques (70 ou 380) et ladite région de contenance de fluide dudit carter (12 ou 312), ladite région de contenant de fluide, l'entrée (18 ou 320), la sortie (20 ou 322) et l'armature (110 ou 420) étant toutes situées axialement d'un côté desdits moyens de barrière (80 ou 390) et lesdits moyens électromagnétiques (70 ou 380) étant situés axialement du côté opposé desdits moyens de barrière;

(b) un corps en matière magnétiquement perméable (90 ou 400) situé dans ladite région de contenance de fluide du carter et traversé par un passage (98 ou 408) pour recevoir ladite partie polaire d'armature (114 ou 424) en relation d'ajustement étroit et de façon mobile; et

(c) des moyens pour définir un circuit magnétique comprenant lesdits moyens électromagnétiques (70 ou 380), ledit corps (90 ou 400), les parties desdits moyens de barrière (80 ou 390) incluses entre lesdits moyens électromagnétiques et ledit corps, ladite partie polaire d'armature (114 ou 424) et un intervalle dans ladite région de contenant de fluide dudit carter entre ladite partie polaire d'armature et lesdits moyens électromagnétiques pour déplacer ladite armature vers lesdits moyens électromagnétiques de façon à fermer ledit intervalle en réponse à l'excitation électrique desdits moyens électromagnétiques.

9. Pompe électromagnétique suivant la revendication 8, caractérisée en ce que lesdits moyens de barrière comprenant une plaque métallique mince (80 ou 390) fixée entre et assurant une fermeture hermétique entre lesdits moyens électromagnétiques (70 ou 380) et ladite région de contenance de fluide dudit carter (12 ou 312), et dans laquelle lesdits moyens électromagnétiques (70 ou 380) comprennent une enveloppe (76 ou 386) en matière magnétiquement perméable présentant une face d'extrémité en contact avec une surface de ladite plaque de barrière, ledit corps (90 ou 400) présente une face d'extrémité en contact avec la surface extérieure de ladite plaque de barrière, et les zones de contact des dites faces d'extrémité de ladite enveloppe d'électroaimant et dudit corps avec ladite surface de ladite plaque ont une dimension suffisante pour minimiser l'intervalle d'air effectif introduit par ladite plaque dans ledit circuit magnétique.

10. Pompe suivant la revendication 8 ou la revendication 9, caractérisée en ce que la longueur axiale dudit passage (98 ou 408) dans ledit corps (90 ou 400) est relativement longue par rapport à la longueur de l'espace défini entre la surface intérieure dudit passage et la surface extérieure de ladite partie polaire d'armature (114 ou 424) afin de minimiser l'intervalle d'air effectif défini entre ces surfaces et introduit dans ledit circuit magnétique.

11. Pompe électromagnétique comprenant un carter (12 ou 312) qui comporte une région intérieure de contenance de fluide, une entrée (18 ou 320) et une sortie (20 ou 322) en communication de fluide avec ladite région, des moyens électromagnétiques (70 ou 380) portés par ledit carter et situés à l'extérieur de ladite région de contenance de fluide, et une armature (110 ou 420) située dans ledit carter pour un mouvement, d'une position de repos, suivant une course de pompage vers l'avant lorsqu'elle est attirée par lesdits moyens électromagnétiques, de manière à refouler le fluide à l'extérieure de ladite région de contenance de fluide par l'intermédiaire de ladite sortie, et pour un mouvement dans une direction opposée suivant une course de retour à ladite positionde repos, caractérisée en ce que:

(a) des moyens à clapet anti-retour (170 ou 468) sont fonctionellement associés à ladite armature (110 ou 420) et situés dans ladite région de contenance de fluide du dit carter (12 ou 312) pour fermer ladite entrée (18 ou 320) lorsque ladite armature est dans ladite position de repos et pour ouvrir ladite entrée après le début du mouvement de ladite armature associé à ladite course de pompage vers l'avant, lesdits

23

moyens à clapet antiretour permettant l'écoulement du fluide dans une direction à partir de ladite entrée et à travers ladite sortie (20 ou 322) et empêchant l'écoulement du fluide dans une direction à partir de ladite sortie et à travers ladite entrée; et

(b) ladite région de contenance de fluide dudit carter et lesdits moyens électromagnétiques (70 ou 380) sont axialement espacés le long dudit axe longitudinal du carter et sont séparés par des moyens de barrière (80 ou 390) en matière imperméable au fluid disposés sensiblement perpendiculairement audit axe longitudinal du carter, ladite région de contenance de fluide, l'entrée (18 ou 320), la sortie (20 ou 322) et l'armature (40 ou 420) étant toutes situés axialement d'un côté desdits moyens de barrière et lesdits moyens électromagnétiques étant situés axialement du côté opposé desdits moyens de barrière.

12. Pompe électromagnétique suivant la revendication 11, caractérisée en ce que lesdits moyens à clapet antiretour (170 ou 468) comprennent un élément de clapet (172 ou 470) porté de façon mobile par ladite armature (110 ou 420), et comprennent en outre:

(a) des premiers moyens de rappel (200 ou 450) pour pousser ladite armature (110 ou 420) vers ladite position de repos;

(b) des deuxièmes moyens de rappel (204 ou 498) pour pousser ledit élément de clapet (172 ou 470) vers une position de fermeture de ladite entrée (18 ou 320); et

(c) la force exercée par lesdits premiers moyens de rappel (200 ou 450) étant supérieure à la force exercée par les dits deuxièmes moyens de rappel (204 ou 498).

13. Pompe électromagnétique suivant la revendication 11 ou la revendication 12, caractérisée en ce que:

(a) ladite région de contenance de fluide comprend une chambre de réception de fluide (314), une première chambre de pompage de fluide (316) en communication de fluide avec celle-ci, une deuxième chambre de pompage de fluide (318) en communication de fluide avec ladite première chambre de pompage, ladite entrée (320) étant en communication de fluide avec ladite chambre de réception et ladite sortie (322) étant en communication de fluide avec ladite deuxième chambre de pompage;

(b) ladite armature (420) est située dans ladite région de contenance de fluide dudit carter (312) et présente une partie polaire (424) située pour l'attraction magnétique par lesdits moyens électromagnétiques (380) et une première partie formant plongeur (434) dans la dite première chambre de pompage (316), pour refouler le fluide à l'extérieur de ladite première chambre et dans ladite deuxième chambre de pompage (318), et une deuxième partie formant plongeur (424) dans ladite deuxième chambre de pompage (318) pour refouler le fluide à l'extérieur de ladite deuxième chambre et à travers ladite sortie (322); ladite armature étant supportée de façon mobile dans ledit carter pour un mouvement à partir d'une position de repos et suivant une course de pompage vers l'avant lorsqu'elle est attirée par lesdits moyens électromagnétiques, de manière à refouler le fluide à partir desdites chambres de pompage (316 et 318) à travers ladite sortie (322), et pour un mouvement dans une direction opposée, suivant une course de retour à ladite position de repos; et

(c) des moyens sont prévus pour définir une circuit magnétique comprenant lesdits moyens électromagnétiques (380) et ladite armature (420) et un intervalle entre ladite partie polaire (424) de ladite armature et lesdits moyens électromagnétiques pour déplacer ladite armature vers lesdits moyens électromagnétiques de manière à fermer ledit intervalle en réponse à l'excitation électrique desdits moyens électromagnétiques.

14. Pompe suivant la revendication 13, caractérisée en ce que lesdits moyens à clapet antiretour (468) sont en relation d'ajustment relativement ètroit avec la partie dudit carter (312) définissant ladite chambre de réception de fluide (314), lesdits moyens à clapet antiretour sont étroitement associés à ladite armature (420) et situés très près de celle-ci, et la partie de ladite armature adjacente auxdits moyens à clapet antiretour est en relation d'ajustment relativement étroit avec ledit carter, de manière à réduire le volume de ladite région intérieure de contenance de fluide entre ladite armature et lesdits moyens à clapet antiretour.

15. Pompe suivant la revendication 14, caractérisée en ce que la dimension de section transversale de la dite partie d'armature adjacente auxdits moyens à clapet antiretour (468) et la dimension de section transversale de la partie associée dudit carter (312) sont en relation mutuelle définissant entre elles un espace de tolérance serré constituant un chemin de fuite pour le fluide de la dite première chambre de pompage (316) à ladite chambre de réception (314) pendant le mouvement de ladite armature suivant ladite cours de retour.

16. Pompe suivant la revendication 13, caractérisée en ce que ledit carter (312) comprend une partie cylindrique (396) adjacente à ladite entrée (320) et comportant une section de premier diamètre intérieur (358) contenant lesdits moyens à clapet antiretour (468) en relation d'ajustement relativement étroit et un section de deuxième diamètre intérieur plus petit (352) contenant ladite première partie formant plongeur (434) de ladite armature (420) en relation d'ajustement relativement étroit, lesdits moyens à clapet antiretour étant étroitement associés et situés très près de ladite armature, de manière à diminuer le volume de ladite région intérieure de contenance de fluide entre la dite armature et lesdits moyens à clapet antiretour.

17. Pompe suivant la revendication 16, caractérisée en ce que le diamètre intérieur de ladite deuxième section de carter (352) et le diamètre extérieur de ladite première partie de plongeur de l'armature (434) sont en relation telle qu'un espace de tolérance serrée est défini entre eux pour constituer un chemin de fuite pour le fluide de la dite première chambre de pompage (316) à ladite chambre de réception (314) pendant le mouvement de ladite course de retour.

18. Pompe suivant l'une quelconque des revendications 13 à 17, comprenant en outre un corps (400) en matière magnétiquement perméable placé dans ladite région de contenance de fluide du carter entre lesdites première et deuxième chambres de pompage de fluide (316 et 318), ledit corps définissant une partie dudit circuit magnétique, le dit corps étant traversé par un passage (408) pour recevoir ladite deuxième partie de plongeur de l'armature (424) dans une relation d'ajustment relativement étroit et de façon mobile, de sorte que ladite armature (428) se déplace dans ledit corps (400) pendant lesdites courses vers l'avant et de retour et ledit circuit magnétique s'effectue par l'intermédiaire dudit corps et de ladite deuxième partie de plongeur (424) de l'armature.

19. Pompe suivant l'une quelconque des revendications 13 ou 18, comprenant en outre des moyens de rappel (450) placés dans ledit carter pour rappeler ladite armature (420) vers ladite position de repos.

20. Pompe suivant la revendication 19, comprenant en outre:

(a) des premiers moyens de rappel (450) pour pousser la dite armature (420) vers ladite position de repos;

(b) des deuxièmes moyens de rappel (498) pour pousser les dits moyens à clapet antiretour (468) vers uneposition de fermeture de ladite entrée (320); et

(c) la force exercée par lesdits permiers moyens de rappel (450) étant supérieure à la force exercée par lesdits deuxièmes moyens de rappel (498).

21. Pompe suivant la revendication 19, comprenant en outre;

(a) des premiers moyens de rappel (450) fonctionellement associés à ladite armature (420) pour assurer l'étanchéité de longue durée de ladite entrée de pompe (320);

(b) des deuxièmes moyens de rappel (498) fonctionellement associés auxdits moyens à clapet antiretour (468) pour assurer la fermeture de courte durée de ladite entrée de pompe (320); et

(c) la force exercée par lesdits deuxièmes moyens de rappel (498) étant inférieure à la force exercée par lesdits premiers moyens de rappel (450);

(d) de sorte que l'échanchéité contre un écoulement de retour est assurée lorsque ladite pompe n'est pas en fonctionnement, tandis que la perte de charge à travers lesdits moyens à clapet antiretour est relativement faible pendant la course de pompage.

Fig. 1.

Fig. 2.

Fig. 3.

Fig. 4.

Fig. 5.

Fig. 6.

Fig. 7.

Fig. 8.

Fig. 9.

Fig. 10.

Fig. 13. Fig. 14. Fig. 11. Fig. 12. Fig. 15. Fig. 16. Fig. 17. Fig. 18.

4

Fig. 20.

Fig. 19.

Fig. 21.

Fig. 22.

0 102 824

Fig. 23.

Fig. 24.

Fig. 25.

Fig. 26.